(19) Europäisches Patentamt
European Patent Office
Office européen des brevets

(11) EP 3 113 239 A1

(12) EUROPÄISCHE PATENTANMELDUNG

(43) Veröffentlichungstag:
**04.01.2017 Patentblatt 2017/01**

(21) Anmeldenummer: **15200813.2**

(22) Anmeldetag: **17.12.2015**

(51) Int Cl.:
***H01L 51/30*** *(2006.01)* *H01L 51/46* *(2006.01)*
*H01L 51/54* *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **03.07.2015 EP 15175358**

(71) Anmelder: **cynora GmbH**
**76646 Bruchsal (DE)**

(72) Erfinder:
- **DANZ, Michael**
**76344 Eggenstein-Leopoldshafen (DE)**
- **ZINK, Daniel**
**76646 Bruchsal (DE)**

(74) Vertreter: **Hoppe, Georg Johannes**
**Darani Anwaltskanzlei**
**Beuckestrasse 20**
**14163 Berlin (DE)**

(54) **ORGANISCHE MOLEKÜLE ZUR VERWENDUNG IN ORGANISCHEN OPTOELEKTRONISCHEN VORRICHTUNGEN**

(57) Die Erfindung betrifft ein organisches Molekül, aufweisend eine Struktur der Formel I

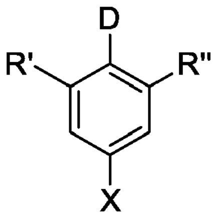

Formel I

oder eine Verbindung enthaltend genau zwei Einheiten gemäß Formel I, die über eine Einfachbindung oder eine Brücke Y miteinander verknüpft sind,
mit
Y =
eine divalente oder trivalente Gruppe;
X =
eine elektronenziehende Einheit, bevorzugt CN oder $CF_3$;
D =
chemische Einheit aufweisend eine Struktur der Formel I-1:

EP 3 113 239 A1

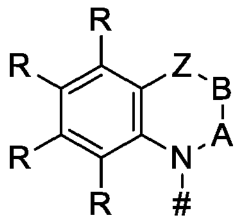

Formel I-1

wobei

# = Anknüpfungspunkt der Einheit gemäß Formel I-1 an die Struktur gemäß Formel I;

A und B = unabhängig voneinander ausgewählt aus der Gruppe bestehend aus $CRR^1$, CR, NR, N, wobei zwischen A und B eine Einfach- oder Doppelbindung vorliegt und zwischen B und Z eine Einfach- oder Doppelbindung vorliegt;

Z = eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination dieser, $-CRR^1$, $-C=CRR^1$, -C=NR, -NR-, -O-, $-SiRR^1-$, -S-,-S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ist.

## Beschreibung

Die Erfindung betrifft rein organische Moleküle und deren Verwendung in organischen lichtemittierenden Dioden (OLEDs) und in anderen organischen optoelektronischen Vorrichtungen.

## Hintergrund

Organische optoelektronische zeichnen sich dadurch aus, dass entweder elektrische Energie in Photonen umgewandelt werden (Organische Lichtemittierende Dioden, OLED oder Lichtemittierende Elektrochemische Zellen, LEEC) oder der umgekehrte Prozess abläuft (Organische Photovoltaik, OPV). Dabei ist es wichtig, dass diese Prozesse möglichst effizient ablaufen. Für den Bereich von OLEDs müssen daher idealerweise Materialien mit möglichst hoher photolumineszenter Quantenausbeute verwendet werden. Begrenzte Effizienzen von OLED-Materialien können durch Verwendung effizienter Materialien, die thermisch aktivierte verzögerte Fluoreszenz (TADF) zeigen, verbessert werden, da im Gegensatz zu rein fluoreszenten Materialien statt 25 % der in einer OLED gebildeten Exzitonen bis zu 100 % der Exzitonen genutzt werden können. Hierbei können auch die entstandenen Triplett-Excitonen in Singulett-Excitonen überführt werden, aus welchem Zustand dann Photonen emittiert werden können. Voraussetzung für eine solche thermische Rückbesetzung ist dabei ein geringer energetischer Abstand zwischen dem niedrigsten angeregten Singulett- ($S_1$) und Triplett-Niveau ($T_1$). Dies kann beispielweise durch Verwendung von Kupfer-(I)-Komplexen (siehe hierzu z. B.: H. Yersin, U. Monkowius, T. Fischer, T. Hofbeck, WO 2010/149748 A1) aber auch durch rein organische Materialien (siehe hierzu z. B.: Q. Zhang et al., J. Am. Chem. Soc. 2012, 134, 14706, WO 2013161437 A1) erreicht werden.

Die intensive Forschung in diesem Bereich zeigt, dass es weiterhin einen großen Bedarf an neuen Materialien gibt. So besteht zum Beispiel nach wie vor der Bedarf an tiefblauen TADF-OLEDs. Bestehende blaue TADF-Materialien zeigen oft hohe Exzitonenlebensdauern, welche schlecht für effiziente und langlebige OLEDs sind. Neben den erwähnten Eigenschaften der Materialien ist für eine Kommerzialisierung ebenso die Zugänglichkeit relevant. Dies schließt die Verfügbarkeit von Synthesebausteinen, als auch den Aufwand für die eigentliche Synthese des funktionellen Materials, insbesondere dessen Aufreinigung mit ein.

## Beschreibung

Die Erfindung stellt eine neue Klasse von Molekülen bereit, die eine Struktur der Formel I aufweisen oder eine Struktur der Formel I haben:

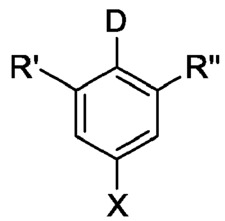

Formel I

oder eine Verbindung enthaltend genau zwei Einheiten gemäß Formel I, die über eine Einfachbindung oder eine über R' oder R" gebildete Brücke Y miteinander verknüpft sind, mit

- Y = eine beliebige divalente oder trivalente Gruppe;
- X = eine elektronenziehende Einheit, bevorzugt CN oder $CF_3$;
- D = chemische Einheit aufweisend eine Struktur der Formel I-1:

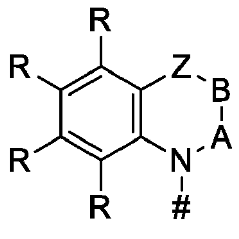

Formel I-1

wobei

# = Anknüpfungspunkt der Einheit gemäß Formel I-1 an die Struktur gemäß Formel I;
A und B = unabhängig voneinander ausgewählt aus der Gruppe bestehend aus $CRR^1$, CR, NR, N, wobei zwischen A und B eine Einfach- oder Doppelbindung vorliegt und zwischen B und Z eine Einfach- oder Doppelbindung vorliegt;
Z = eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination dieser, $-CRR^1$, $-C=CRR^1$, -C=NR, -NR-, -O-, $-SiRR^1-$, -S-, - S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ist;
wobei jedes R und $R^1$ bei jedem Auftreten gleich oder verschieden ist H, Deuterium, Azid ($N_3^-$), F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination dieser Systeme, oder eine quervernetzbare Einheit QE ist, die durch säurekatalytische, thermische oder UV-Quervernetzungsverfahren in Anwesenheit oder Abwesenheit eines Photoinitiators oder durch Mikrowellenstrahlung quervernetzt werden kann; dabei können zwei oder mehrere dieser Substituenten R und $R^1$ auch miteinander ein mono- oder polycyclisches, aliphatisches, aromatisches und/oder benzoannelliertes Ringsystem bilden;
$R^2$ bei jedem Auftreten gleich oder verschieden ist H, Deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, $C\equiv C$, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;
$R^3$ bei jedem Auftreten gleich oder verschieden ist H, Deuterium, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;
R' = Anknüpfungsposition für die zweite Einheit der Formel I oder ausgewählt aus der Gruppe bestehend aus Y, H, $N(R^4)_2$, $OR^4$, eine lineare Alkyl- oder Alkoxygruppe mit 1 bis 40 C-Atomen oder eine verzweigte oder cyclische Alkyl- oder Alkoxygruppe mit 3 bis 40 C-Atomen, die jeweils mit einem oder mehreren Resten $R^4$ substituiert sein kann, oder ein aromatisches oder heteroaromatisches Ringsystem mit 5 bis 60 aromatischen Ringatomen, das jeweils durch einen oder mehrere Reste $R^4$ substituiert sein kann;
R" = Anknüpfungsposition für die zweite Einheit der Formel I oder ausgewählt aus der Gruppe bestehend aus Y, $N(R^4)_2$, $OR^4$, eine lineare Alkyl- oder Alkoxygruppe mit 1 bis 40 C-Atomen oder eine verzweigte oder cyclische Alkyl- oder Alkoxygruppe mit 3 bis 40 C-Atomen, die jeweils mit einem oder mehreren Resten $R^4$ substituiert sein kann, oder ein aromatisches oder heteroaromatisches Ringsystem mit 5 bis 60 aromatischen Ringatomen, das jeweils durch einen oder mehrere Reste $R^4$ substituiert sein kann;
mit der Maßgabe, dass wenn R' gleich Y ist, R" ungleich Y ist und wenn R" gleich Y ist, R' ungleich Y ist;
und mit der Maßgabe, dass wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;

$R^4$ bei jedem Auftreten gleich oder verschieden ist H, Deuterium, $N(R^5)_2$, $Si(R^5)_3$, eine lineare Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^5$ substituiert sein kann, oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoanelliertes Ringsystem bilden;

$R^5$ bei jedem Auftreten gleich oder verschieden ist H, Deuterium, oder ein aliphatischer, aromatischer und/oder heteroaromatischer Kohlenwasserstoffrest mit 1 bis 20 C-Atomen; dabei können zwei oder mehrere Substituenten $R^5$ auch miteinander ein mono- oder polycyclisches, aliphatisches Ringsystem bilden.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt N, O, und/oder S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen dieser Gruppen.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$ bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Die chemische Einheit D der erfindungsgemäßen Moleküle weist Donoreigenschaften auf. Was unter Donor- bzw. Akzeptoreigenschaften verstanden wird, ist dem Fachmann prinzipiell bekannt. Die chemische Einheit D ist in einer bevorzugten Ausführungsform elektronenschiebend. Sie weist einen +M-Effekt (positiven mesomeren Effekt) auf. Insbesondere weisen geeignete Donorsubstituenten ein Atom mit einem freien Elektronenpaar auf, wie zum Beispiel ein

N-, O- oder S-Atom. Hierbei sind 5-Ring-Heteroarylgruppen mit genau einem Heteroringatom bevorzugt. An diese können auch weitere Arylgruppen ankondensiert sein. Hierbei sind insbesondere Carbazolgruppen bzw. Carbazolderivate bevorzugt. Weitere geeignete Donorsubstituenten sind Phenoxazingruppen bzw. Phenoxazinderivate. Bei letzteren kann der Sauerstoff des Phenoxazins beispielsweise durch $-CRR^1$, $-C=CRR^1$, -C=NR, -NR-, $-SiRR^1-$, -S-, -S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ersetzt sein.
In einer bevorzugten Ausführungsform übt der elektronenziehende Rest X einen -M-Effekt (negativen mesomeren Effekt) oder einen -I-Effekt (negativen induktiven Effekt) aus. Der Rest X ist entsprechend ein Akzeptorsubstiutent. Geeignete Akzeptorsubstituenten sind insbesondere Cyanogruppen oder $CF_3$.

Die erfindungsgemäßen Moleküle weisen in ortho-Stellung zum Donor am Aromaten einen Substituenten auf. Dies ermöglicht eine effektive Trennung von HOMO und LUMO des organischen Moleküls.

Die erfindungsgemäßen Moleküle zeigen thermisch aktivierte verzögerte Fluoreszenz und emittieren bevorzugt im tiefblauen Bereich des sichtbaren Spektrums.
Die Verwendung der erfindungsgemäßen Moleküle in einer optoelektronischen Vorrichtung, beispielsweise einer OLED, führt zu höheren Effizienzen der Vorrichtung. Weiterhin lassen sich OLEDs im tiefblauen Farbspektrum realisieren. Entsprechende OLEDs weisen eine höhere Stabilität als OLEDs mit bekannten Emittermaterialien und vergleichbarer Farbe.

Bei den quervernetzbaren Einheiten QE handelt es sich in einer Ausführungsform um eine Verbindung ausgewählt aus der Gruppe bestehend aus Oxetanen, Alkinen und Aziden, insbesondere für eine Clickreaktion, sowie folgenden Alkenderivaten:

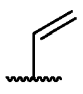
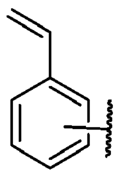
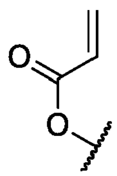
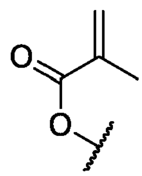
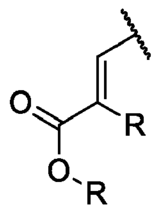
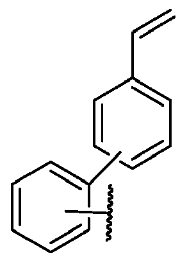

In einer alternativen Ausführungsform handelt es sich bei Z um eine kovalente Einfachbindung oder eine divalente organische Brücke ausgewählt aus substituierten und unsubstituierten Alkylen- (auch verzweigt oder zyklisch), Alkenylen-, Alkinylen-, Arylen- und Heteroarylen-Gruppen, O, NR, $C=CR_2$, C=NR, $SiR_2$ S, S(O), $S(O)_2$, BR, PR, P(O)R, wobei auch Kombinationen dieser Einheiten möglich sind (z.B. durch O unterbrochene Alkylen-(auch verzweigt oder zyklisch), Alkenylen-, Alkinylen-, Arylen- und Heteroarylen-Gruppen).

In einer bevorzugten Ausführungsform ist D unabhängig voneinander jeweils eine Donorgruppe mit elektronenschiebenden Eigenschaften, die ausgewählt aus der Gruppe bestehend aus substituierten und unsubstituierten Carbazol, substituierten und unsubstituierten Indol, substituierten und unsubstituierten Indolin, substituierten und unsubstituierten Dihydroacridin, substituierten und unsubstituierten Benzimidazol, substituierten und unsubstituierten 2,3,4,9-Tetrahydrocarbazol, substituierten und unsubstituierten 1,2,3,4-Tetrahydrochinolin, substituierten und unsubstituierten Phenothiazin, substituierten und unsubstituierten Phenoxazin, substituierten und unsubstituierten Dihydrophenazin, substituierten und unsubstituierten Spiroverbindungen.

In einer Ausführungsform des organischen Moleküls weist die Donorgruppe mit elektronenschiebenden Eigenschaften der Formel I-1 eine Struktur der Formel II auf:

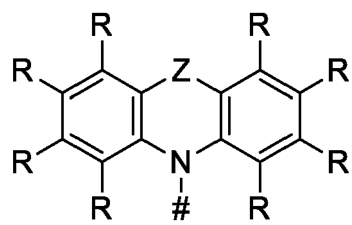

Formel II

wobei für #, Z und R die oben in Verbindung mit Formel I genannten Definitionen gelten.

Die Donorgruppe mit elektronenschiebenden Eigenschaften der Formel I-1 kann in einer Ausführungsform eine

Struktur der Formel III aufweisen:

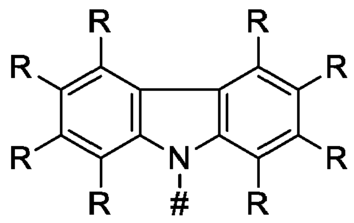

Formel III

wobei für # und R die oben in Verbindung mit Formel I genannten Definitionen gelten.

Beispiele für erfindungsgemäße Donoren:

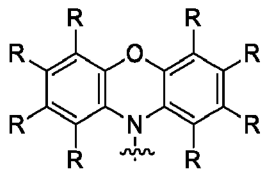
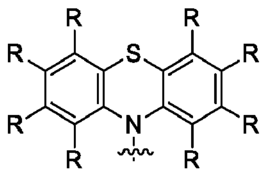
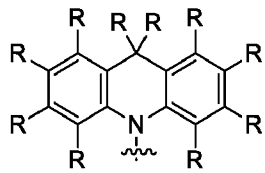
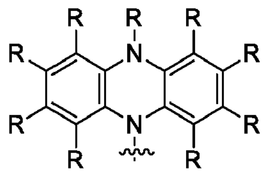
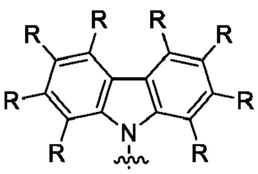
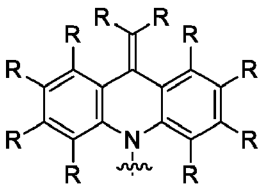
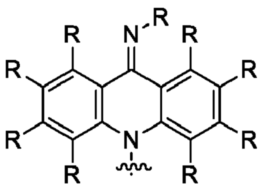
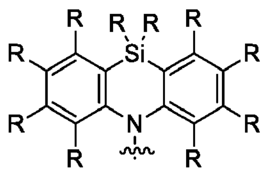
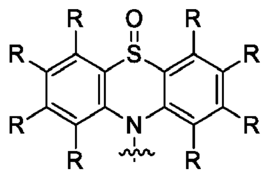
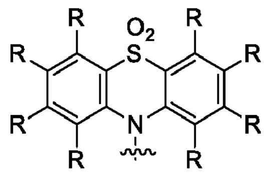
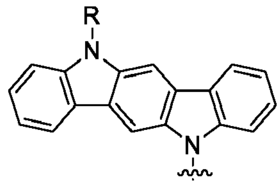
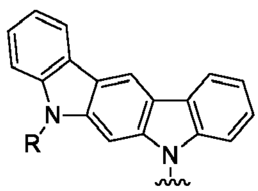
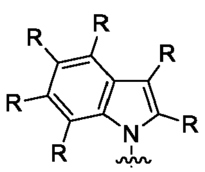
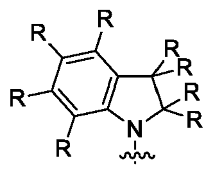
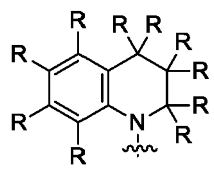
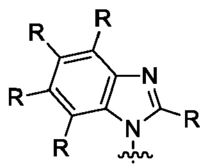
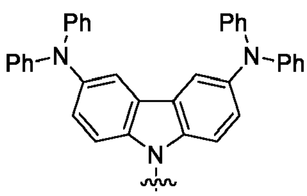
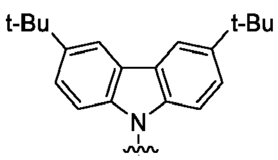
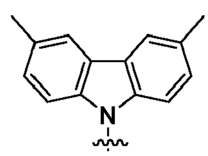
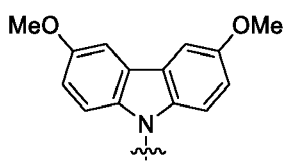

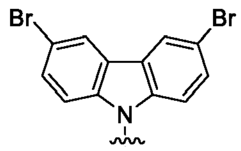
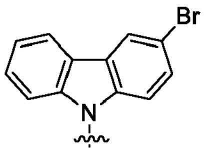
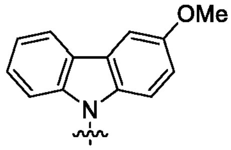
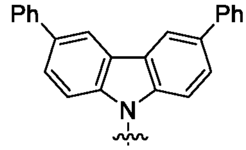

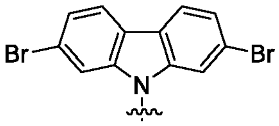
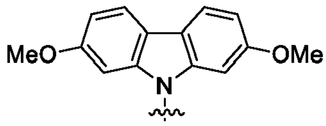
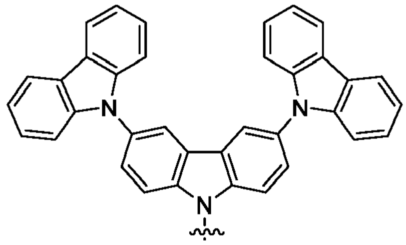

Die akzeptierende Einheit X der Formel I ist in einer Ausführungsform gleich CN, in einer weiteren Ausführungsform gleich $CF_3$. In einer weiteren Ausführungsform der Erfindung ist der Rest R' der Formel I ein Wasserstoffatom, also H.

Die Brücke Y ist eine organische Brücke, die in einer Ausführungsform eine substituierte oder unsubstituierte Alkylen-, Alkenylen-, Alkinylen-, Arylen- oder Heteroarylen-Gruppe oder eine Kombination dieser, oder-O-, -NR-, $-C=CR_2$, -C=NR, $-SiR_2$- -S-, -S(O)-, $-S(O)_2$-, durch O unterbrochene Alkyl- (auch verzweigt oder zyklisch), Heteroalkyl-, Aryl-, Heteroaryl-, AlkenylGruppen, Phenyl- und substituierte Phenyleinheiten ist; wobei für R die oben genannten Definitionen gelten.

Die Brücke Y kann beispielsweise ausgewählt sein aus einer der Strukturen der Formeln IV bis X:

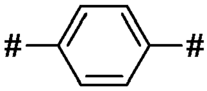
IV

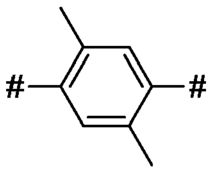
V

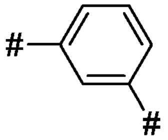
VI

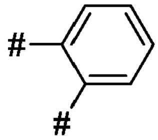
VII

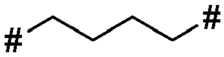
VIII

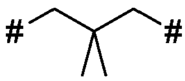
IX

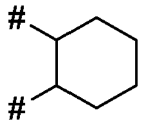
X

mit

# = Anknüpfungspunkt der Brücke Y an die Struktur gemäß Formel I.

In einer Ausführungsform weisen die erfindungsgemäßen Moleküle eine Struktur der Formel XI-a, XI-b oder XI-c auf:

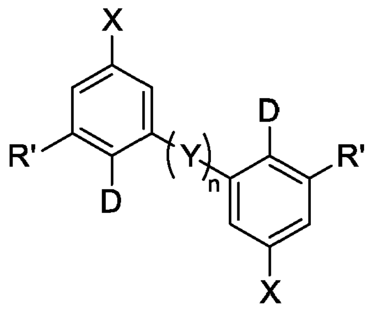

Formel XI-a

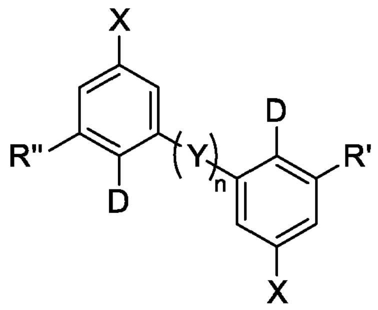

Formel XI-b

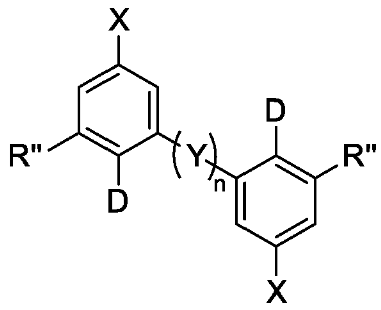

Formel XI-c

mit n = 0 oder 1;
und wobei für D, R', R", X und Y die oben genannten Definitionen gelten.

In einem weiteren Aspekt betrifft die Erfindung ein Verfahren zur Herstellung eines erfindungsgemäßen organischen Moleküls der hier beschriebenen Art (mit einer eventuellen Folgeumsetzung).

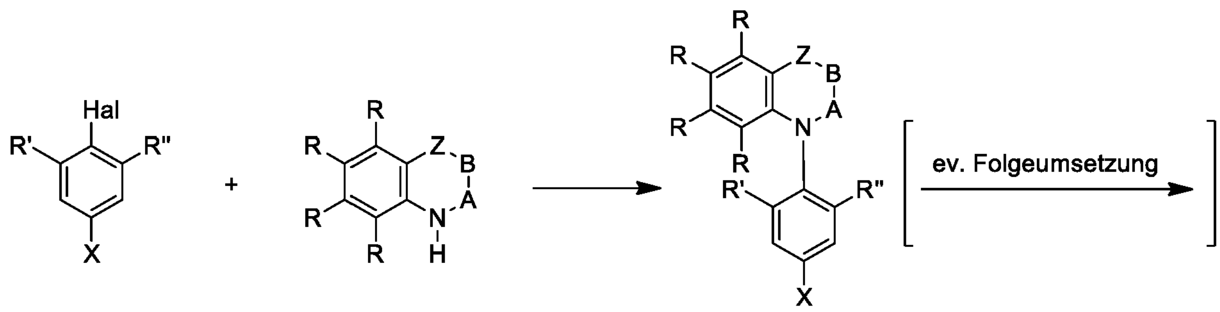


Hierbei wird ein Stickstoffheterozyklus im Sinne einer nukleophilen aromatischen Substitution mit einem Arylhalogenid, bevorzugt einem Arylfluorid umgesetzt. Typische Bedingungen beinhalten die Verwendung einer Base wie beispielweise tribasisches Kaliumphosphat oder Natriumhydrid in einem aprotisch polarem Lösungsmittel wie beispielweise Dimetylsulfoxid (DMSO) oder N,N-Dimethylformamid (DMF).
Eine alternative Syntheseroute beinhaltet die Einführung eines Stickstoffheterozyklus über eine Kupfer- oder Palladiumkatalysierte Kupplung mit einem Arylhalogenid oder Arylpseudohalogenid, bevorzugt ein Arylbromid, ein Aryliodid, Aryltriflat oder ein Aryltosylat. Die beschriebenen Herstellungsweisen können dabei sowohl die letzte synthetische Umsetzung darstellen als auch ein Vorläufermolekül liefern, welches durch Folgeumsetzungen, beispielsweise durch Änderung der Reste R, R' bzw. R", zum erfindungsgemäßen Molekül umgesetzt werden kann.
Ein weiterer Aspekt der Erfindung betrifft die Verwendung eines organischen Moleküls der hier beschriebenen Art als lumineszierender Emitter oder als Absorber, und/oder als Hostmaterial und/oder als Elektronentransportmaterial, und/oder als Lochinjektionsmaterial, und/oder als Lochblockiermaterial in einer organischen optoelektronischen Vorrichtung.
Im Rahmen einer derartigen Verwendung ist die organische optoelektronische Vorrichtung insbesondere ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

Bei der Verwendung beträgt der Anteil des organischen Moleküls an der Emissionsschicht in einer organischen

optoelektronischen Vorrichtung, insbesondere in OLEDs, 1 % bis 99 %, insbesondere 5 % bis 80 % (Gew%). In einer alternativen Ausführungsform beträgt der Anteil des organischen Moleküls an der Emissionsschicht 100%.

In einer Ausführungsform weist die lichtemittierende Schicht neben dem erfindungsgemäßen organischen Molekül ein Hostmaterial auf, dessen Triplett (T1)- und Singulett (S1)-Energieniveaus energetisch höher liegen als die Triplett (T1)- und Singulet (S1)-Energieniveaus des organischen Moleküls.

In einem weiteren Aspekt betrifft die Erfindung eine organische optoelektronische Vorrichtung, aufweisend ein organisches Molekül der hier beschriebenen Art, insbesondere ausgeformt als eine Vorrichtung ausgewählt aus der Gruppe bestehend aus organischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine derartige organische optoelektronische Vorrichtung weist in einer Ausführungsform auf:

- ein Substrat,
- eine Anode und
- eine Kathode, wobei insbesondere die Anode oder die Kathode unmittelbar auf das Substrat aufgebracht sind, und
- mindestens eine lichtemittierende Schicht, die zwischen Anode und Kathode angeordnet ist und die das erfindungsgemäße organische Molekül aufweist.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier und im Folgenden wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED einen dem Fachmann bekannten invertierten Aufbau auf.

Gemäß einer weiteren Ausführungsform weist das organische Bauelement, insbesondere eine OLED einen dem Fachmann bekannten gestapelten Aufbau auf. Hierdurch kann die Erzeugung von Mischlicht ermöglicht werden. Weiterhin können bei praktisch gleicher Effizienz und identischer Leuchtdichte signifikant längere Lebensdauern im Vergleich zu üblichen OLEDs erzielt werden.

Über den Elektroden und den organischen Schichten kann weiterhin noch eine Verkapselungsanordnung angeordnet sein. Die Verkapselungsanordnung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, Siliziumwafer oder jedes andere geeignete feste oder flexible, optional durchsichtige Material dienen.

Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methyl-phenyl)amino}phenyl]-N-phenylamino]biphenyl), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N,N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPNPB, MeO-TPD, HAT-CN oder TrisPcz (9,9'-Diphenyl-6-(9-phenyl-9H-carbazol-3-yl)-9H,9'H-3,3'-bicarbazol) dienen.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), trisPcz (9,9'-Diphenyl-6-(9-phenyl-9H-carbazol-3-yl)-9H,9'H-3,3'-bicarbazol) oder DCB (N,N'-Dicarbazolyl-1,4-dimethylbenzol) dienen.

Die Emitter-Schicht EML enthält oder besteht aus Emittermaterial oder einer Mischung aufweisend mindestens zwei Emittermaterialien und optional aus einem oder mehreren Hostmaterialien. Geeignete Hostmaterialien sind beispielsweise mCP, TCTA, 2-TNATA, mCBP, CBP (4,4'-Bis-(N-carbazolyl)-biphenyl) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BA1q), Nbphen (2,9-Bis(naphthalen-2-yl)-4,7-diphenyl-1,10-phenanthrolin), A1q3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen.

Die Elektronentransportschicht ETL kann beispielsweise Materialien auf Basis von $AlQ_3$, TSPO1, BPyTP2 (2,7-Di(2,2'-bipyridin-5-yl)triphenyl) oder BTB (4,4'-Bis-[2-(4,6-diphenyl-1,3,5-triazinyl)]-1,1'-biphenyl) aufweisen.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100-200 nm. Besonders bevorzugt werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

Dem Fachmann ist hierbei bekannt, welche Kombinationen der Materialien für eine optoelektronische Vorrichtung enthaltend ein erfindungsgemäßes organisches Molekül zu nutzen sind.

In einer Ausführungsform der erfindungsgemäßen organischen optoelektronischen Vorrichtung ist das erfindungsgemäße organische Molekül als Emissionsmaterial in einer lichtemittierenden Schicht eingesetzt, wobei es entweder als Reinschicht oder in Kombination mit einem Matrixmaterial eingesetzt ist.

Der Anteil des erfindungsgemäßen organischen Moleküls an der Emissionsschicht beträgt in einer weiteren Ausführungsform in einer lichtemittierenden Schicht in optischen Licht emittierenden Vorrichtungen, insbesondere in OLEDs, zwischen 5% und 80%. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und

elektronentransportierende Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein Hostmaterial aufweist, dessen Triplett (T1)-und Singulett (S1)-Energieniveaus energetisch höher liegen als die Triplett (T1)- und Singulett (S1)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

Erfindungsgemäß ist auch ein lichtemittierendes Material, aufweisend ein erfindungsgemäßes organisches Molekül und ein Hostmaterial, wobei die Triplett (T1)- und Singulett (S1)-Energieniveaus des Hostmaterials energetisch höher liegen als die Triplett (T1)- und Singulett (S1)-Energieniveaus des organischen Moleküls, und wobei das lichtemittierende Material Fluoreszenz oder thermisch aktivierte verzögerte Fluoreszenz emittiert, und einen deltaE(S1-T1)-Wert zwischen dem untersten angeregten Singulett (S1)- und dem darunter liegenden Triplett (T1)-Zustand von kleiner als 3000 $cm^{-1}$ aufweist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

## Beispiele

### Allgemeine Arbeitsvorschriften: Photophysikalische Messungen

#### *Vorbehandlung von optischen Gläsern*

Nach jeder Benutzung wurden die optischen Gläser (Küvetten und Substrate aus Quarzglas, Durchmesser: 1cm) gereinigt. Je dreimaliges Spülen mit Dichlormethan, Aceton, Ethanol, demineralisiertem Wasser. Einlegen in 5% Hellmanex-Lösung für 24h, gründliches Ausspülen mit demineralisiertem Wasser, zum Trocknen wurden die optischen Gläser mit Stickstoff abgeblasen.

#### *Probenvorbereitung: Lösungen*

1-2 mg der Probe wurden in 100 ml des jeweiligen Lösemittels gelöst, Konzentration $10^{-5}$ mol/L. Die Küvette wurde luftdicht verschlossen und 10 min. entgast.

#### *Probenvorbereitung, Film: Spin-Coating*

Gerät: Spin150, SPS euro.

Die Probenkonzentration entsprach 10mg/ml, angesetzt in Toluol oder Chlorbenzol. Programm: 1) 3 s bei 400 U/min; 2) 20 Sek. bei 1000 U/min bei 1000 Upm/ s. 3) 10s bei 4000 U/min bei 1000 Upm/ s. Die Filme wurden nach dem Beschichten für 1 min bei 70 °C an Luft auf einer Präzisionsheizplatte von LHG getrocknet.

#### *Absorptionsspektroskopie*

Lösungen:

UV-VIS-Spektren wurden auf einem Gerät der Firma Thermo Scientific, Modell Evolution 201 aufgenommen. (Siehe Probenvorbereitung: Lösungen)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

***Quanteneffizienzbestimmung***

Die Messung der Photolumineszenzquantenausbeute erfolgte mittels eines *Absolute PL Quantum Yield Measurement* C9920-03G-Systems der Firma *Hamamatsu Photonics*. Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned*-) CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.

Das Emissionsmaximum wird in nm, die Quantenausbeute Φ in % und die CIE-Farbkoordinaten als x,y-Werte angegeben.

PLQY wurde für Polymerfilme, Lösungen und Pulverproben nach folgendem Protokoll bestimmt:

Als Referenzmaterial dient Anthracene in Ethanol mit bekannter Konzentration. Es wurde zuerst das Absorbtionsmaximum der Probe bestimmt und mit diesem angeregt.
Anschließend wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Berechnungen nach der Dichtefuntionaltheorie**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D. , J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F. ; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, a development of University of Karlsruhe and Forschungszentrum Karlsruhe GmbH, 1989-2007, TURBOMOLE GmbH, since 2007; available from http://www. turbomole.com) durchgeführt.

**Beispiel 1**

Synthese (Stufe 1):

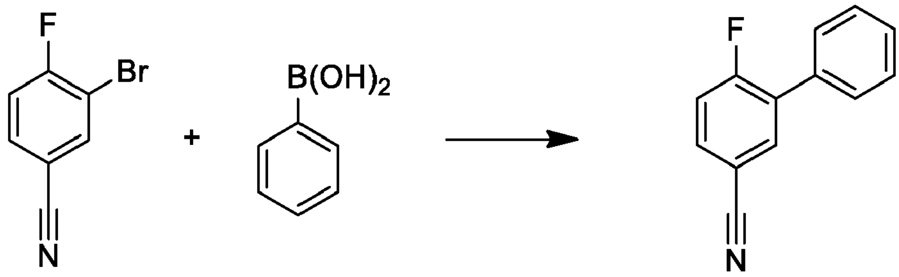

3-Brom-4-fluorbenzonitril (125 mmol), Phenylboronsäure (188 mmol), Palladiumacetat (2.5 mmol), 2-Dicyclohexylphosphino-2‘,6‘-dimethoxy-1,1'biphenyl (SPhos; 5 mmol) und tribasisches Kaliumphosphat (250 mmol) werden unter Stickstoff in 200 mL Toluol suspendiert. Nach Zugabe von 10 mL entgastem Wasser wird die Reaktionsmischung 20 min mit Stickstoff gespült. Nach Rühren für 18 h bei 110 °C und abkühlen auf Raumtemperatur wird die Reaktionslösung filtriert und der Feststoff mit Essigsäureethylester gewaschen. Das Filtrat wird über $MgSO_4$ getrocknet. Nach Entfernen des Lösungsmittels wird das Rohprodukt aus n-Hexan umkristallisiert. 3-Phenyl-4-fluorobenzonitril (15.9 g, 80.6 mmol, 64 %) wird als weißer Feststoff erhalten.

$^1$H NMR (500 MHz, Chloroform-*d*) δ 7.77 (dd, *J* = 7.1, 2.2 Hz, 1H), 7.64 (ddd, *J* = 8.5, 4.5, 2.2 Hz, 1H), 7.54 - 7.42 (m, 5H), 7.27 (dd, J = 9.9, 8.5 Hz, 1H).

Stufe 2:

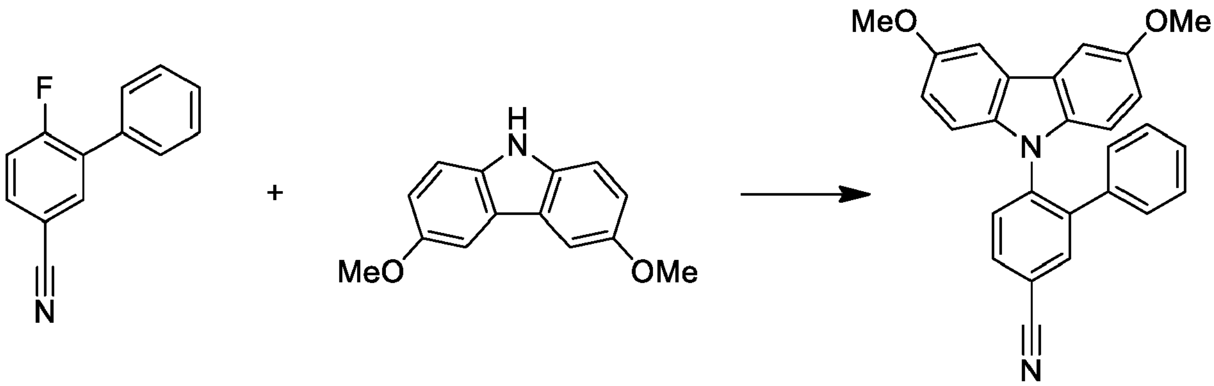

**1**

3-Phenyl-4-fluorobenzonitril (65.9 mmol), 3,6-Dimethoxycarbazol (65.9 mmol) und tribasisches Kaliumphosphat (132 mmol) werden unter Stickstoff in DMSO (120 mL) suspendiert und bei 110 °C gerührt (16 h). Anschließend wird die Reaktionsmischung in 700 mL Wasser gegeben und 1 h gerührt. Der Feststoff wird abfiltriert und mit Wasser (1 L) gewaschen. Anschließend wird der Feststoff im Vakuum bei 50 °C getrocknet. Das Rohprodukt wird durch Umkristallisation aus Toluol gereinigt. Das Produkt **1** (23.1 g, 57.1 mmol, 87%) wird als weißer Feststoff erhalten.

$^1$H NMR (500 MHz, Chloroform-*d*) δ 7.95 (d, *J* = 1.9 Hz, 1H), 7.80 (dd, *J* = 8.2, 2.0 Hz, 1H), 7.64 (d, *J* = 8.1 Hz, 1H), 7.45 (d, *J* = 2.4 Hz, 2H), 7.10 - 7.02 (m, 3H), 7.02 - 6.98 (m, 2H), 6.92 (dd, *J* = 8.9, 0.7 Hz, 2H), 6.89 (dd, *J* = 8.9, 2.3 Hz, 2H), 3.90 (s, 6H).

Das Absorptionsspektrum von **1** als Lösung in 2-Methyltetrahydrofuran ist in Figur 1 gezeigt.

Die Filmemission von **1** (10 % in PMMA) ist Figur 2 zu entnehmen. Das Emissionsmaximum liegt bei 443 nm. Die Photolumineszenzquantenausbeute (PLQY) beträgt 56%.

Die Dichtefunktionaltheorie-Rechnungen ergeben eine Singulett(S1)-Energie von 2.86 eV und eine Triplett(T1)-Energie von 2.59 eV.

Die berechneten Grenzorbitale des Grundzustands von 1 sind in Figur 3 gezeigt.

**Beispiel 2**

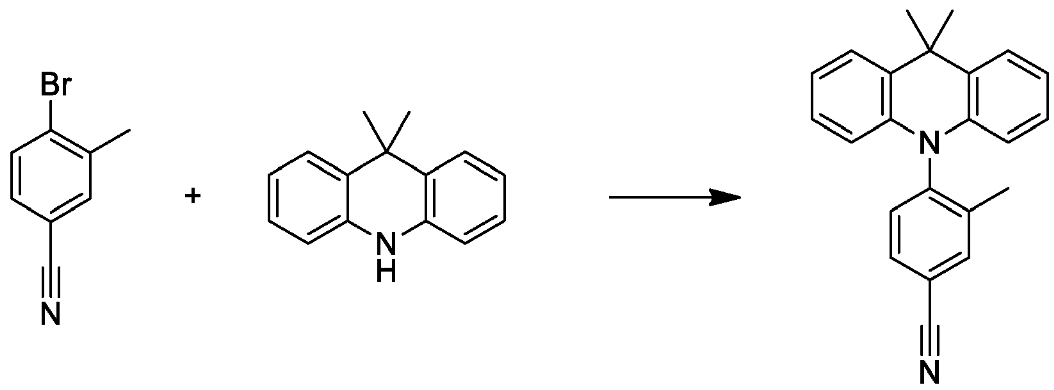

2

9,10-Dihydro-9,9-dimethylacridin (10 mmol), 4-Brom-3-metyhlbenzonitril (15 mmol) und Palladiumacetat (0.5 mmol) werden unter Stickstoff in 50 mL Toluol suspendiert. Nachdem 30 min Stickstoff durch die Reaktionsmischung geleitet wurde werden Tri-*tert*.-butylphosphin (0.75 mmol) und Natrium-*tert*.-butylat (15 mmol) zugegeben und 2.5 h unter Rühren refluxiert. Nachdem im Anschluss 16 h bei RT gerührt wurde, werden 50 mL Wasser zugegeben und zweimal mit je 50 mL Essigsäureethylester extrahiert, Die vereinigten organischen Phasen werden mit gesättigter NaCl-Lösung gewaschen und über $MgSO_4$ getrocknet. Nach Entfernen des Lösungsmittels wird der Rückstand aus Ethanol umkristallisiert. Es werden 1.6 g (4.9 mmol, 49%) des Produktes **2** erhalten.

$^1$H NMR (500 MHz, Chloroform-*d*) δ 7.81 (d, *J* = 1.9 Hz, 1H), 7.75 (dd, *J* = 8.1, 1.9 Hz, 1H), 7.53 - 7.46 (m, 2H), 7.38 (d, *J* = 8.0 Hz, 1H), 7.04 - 6.91 (m, 4H), 6.07 - 5.97 (m, 2H), 2.14 (s, 3H), 1.74 (s, 3H), 1.68 (s, 3H).

Das Absorptionsspektrum von **2** als Lösung in 2-Methyltetrahydrofuran ist in Figur 4 gezeigt. Die Filmemission von **2** (10 % in PMMA) ist Figur 5 zu entnehmen. Das Emissionsmaximum liegt bei 441 nm. Die Photolumineszenzquantenausbeute (PLQY) beträgt 55%.

**Beispiel 3**

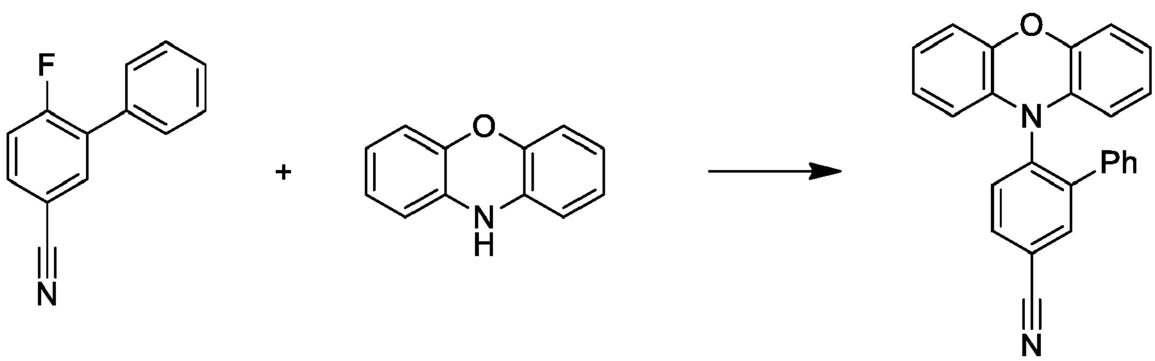

3

3-Phenyl-4-fluorobenzonitril (10 mmol), 2,3:5,6-Dibenzo-1,4-oxazin (10 mmol) und tribasisches Kaliumphosphat (20 mmol) werden unter Stickstoff in DMSO (20 mL) suspendiert und bei 125 °C gerührt (14 h). Anschließend wird die Reaktionsmischung in 400 mL ges. Natriumchlorid-Lösung gegeben und mit Dichlormethan (3 x 150 mL) extrahiert. Die vereinigten organischen Phasen werden mit ges. Natriumchlorid-Lösung gewaschen (2 x 150 mL), getrocknet über Magnesiumsulfat und das Lösemittel anschließend entfernt. Das Rohprodukt wurde schließlich durch Umkristallisation aus Toluol gereinigt. Das Produkt **3** wurde als gelber Feststoff erhalten.

$^1$H NMR (500 MHz, Chloroform-*d*) δ 7.91 (d, *J* = 1.9 Hz, 1H), 7.85 (dd, *J* = 8.1, 2.0 Hz, 1H), 7.57 (d, *J* = 8.1 Hz, 1H), 7.35-7.2.9 (m, 5H), 6.65 - 6.59 (m, 4H), 6.59-6.54 (m, 2H), 5.90-5.83 (m, 2H).

Die Filmemission von 3 (10 % in PMMA) ist Figur 6 zu entnehmen. Das Emissionsmaximum liegt bei 494 nm. Die Photolumineszenzquantenausbeute (PLQY) beträgt 65%.

**Beispiel 4**

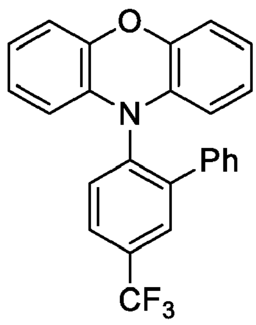

**4**

$^1$H NMR (500 MHz, Chloroform-*d*) δ 7.88 (s, 1H), 7.83 (d, *J* = 7.6 Hz, 1H), 7.57 (d, *J* = 8.2 Hz, 1H), 7.39 - 7.27 (m, 5H), 6.69 - 6.51 (m, 6H), 5.89 (d, *J* = 7.6 Hz, 2H).

Die Filmemission von **4** (10 % in PMMA) ist Figur 7 zu entnehmen. Das Emissionsmaximum liegt bei 466 nm. Die Photolumineszenzquantenausbeute (PLQY) beträgt 31%. Die Emissionslebensdauer beträgt 51 μs.

**Beispiel 5**

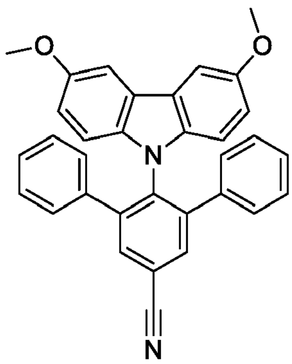

**5**

$^1$H NMR (500 MHz, Chloroform-d) δ 7.87 (s, 2H), 7.30 (d, J = 2.4 Hz, 2H), 7.05 - 7.00 (m, 2H), 6.99 - 6.94 (m, 4H), 6.94 - 6.89 (m, 4H), 6.77 (dd, J = 8.8, 2.4 Hz, 2H), 6.72 (d, J = 8.8 Hz, 2H), 3.84 (s, 6H).

Die Filmemission von 5 (10 % in PMMA) ist Figur 8 zu entnehmen. Das Emissionsmaximum liegt bei 449 nm. Die Photolumineszenzquantenausbeute (PLQY) beträgt 65 %.

**Beispiel 6**

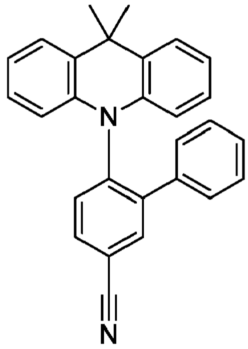

**6**

$^1$H NMR (500 MHz, Chloroform-*d*) δ = 7.93 (d, 1H), 7.90 (dd, 1H), 7.48 (d, 1H), 7.38 (dd, 2H), 7.21 - 7.13 (m, 5H), 6.97 (dt, 2H), 6.91 (dt, 2H), 6.15 (dd, 2H), 1.80 (s, 3H), 1.04 (s, 3H) ppm.

Die Filmemission von **6** (10 % in PMMA) ist Figur 9 zu entnehmen. Das Emissionsmaximum liegt bei 456 nm. Die Photolumineszenzquantenausbeute (PLQY) beträgt 60%.

**Beispiel 7**

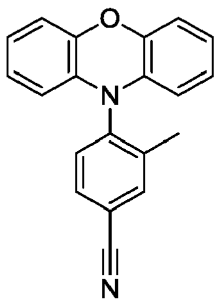

**7**

$^1$H NMR (500 MHz, Chloroform-d) δ 7.77 (d, J = 1.8 Hz, 1H), 7.72 (dd, J = 8.0, 1.9 Hz, 1H), 7.43 (d, J = 8.0 Hz, 1H), 6.76 - 6.65 (m, 4H), 6.60 (td, J = 7.6, 1.7 Hz, 2H), 5.71 (dd, J = 7.9, 1.4 Hz, 2H), 2.29 (s, 3H).

Die Filmemission von **7** (10 % in PMMA) ist Figur 10 zu entnehmen. Das Emissionsmaximum liegt bei 477 nm. Die Photolumineszenzquantenausbeute (PLQY) beträgt 71%.

**Beispiel 8**

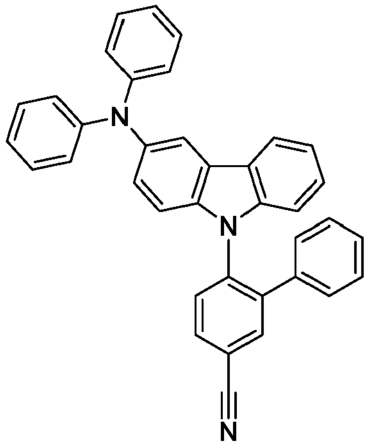

**8**

$^1H$ NMR (500 MHz, Chloroform-*d*) δ = 7.98 (d, 1H), 7.89 (d, 1H), 7.85 (dd, 1H), 7.77 (d, 1H), 7.72 (d, 1H), 7.31 - 7.27 (m, 1H), 7.23 - 7.16 (m, 5H), 7.14 - 7.11 (m, 1H), 7.08 - 7.01 (8H), 6.98 - 6.94 (m, 4H), 6.88 (d, 1H) ppm.

Die Filmemission von **8** (10 % in PMMA) ist Figur 11 zu entnehmen. Das Emissionsmaximum liegt bei 462 nm. Die Photolumineszenzquantenausbeute (PLQY) beträgt 40%.

**Beispiel 9**

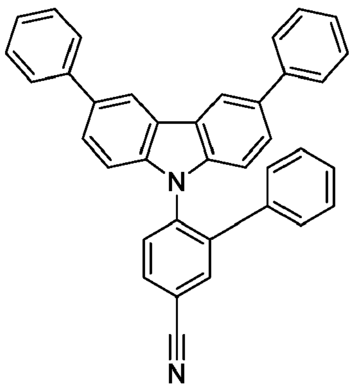

**9**

$^1H$ NMR (500 MHz, Chloroform-*d*) δ = 8.30 (d, 2H), 8.02 (d, 1H), 7.87 (d, 1H), 7.72 (d, 1H), 7.68 (dd, 4H), 7.55 (dd, 2H), 7.47 (t, 4H), 7.35 (t, 2H), 7.11 - 7.04 (m, 7H) ppm.

Die Filmemission von **9** (10% in PMMA) ist Figur 12 zu entnehmen. Das Emissionsmaximum liegt bei 404 nm. Die Photolumineszenzquantenausbeute (PLQY) beträgt 28 %.

**Beispiel 10**

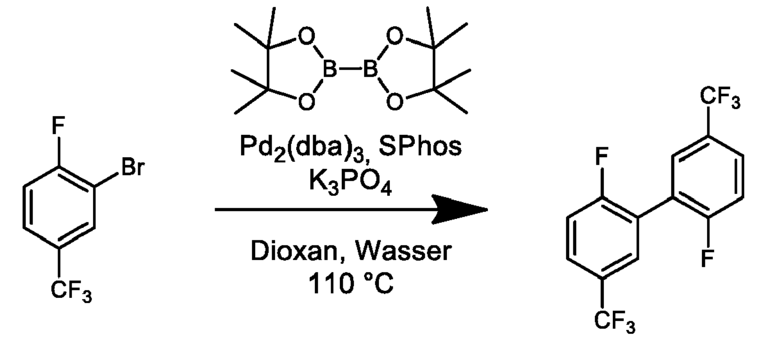


**10a**

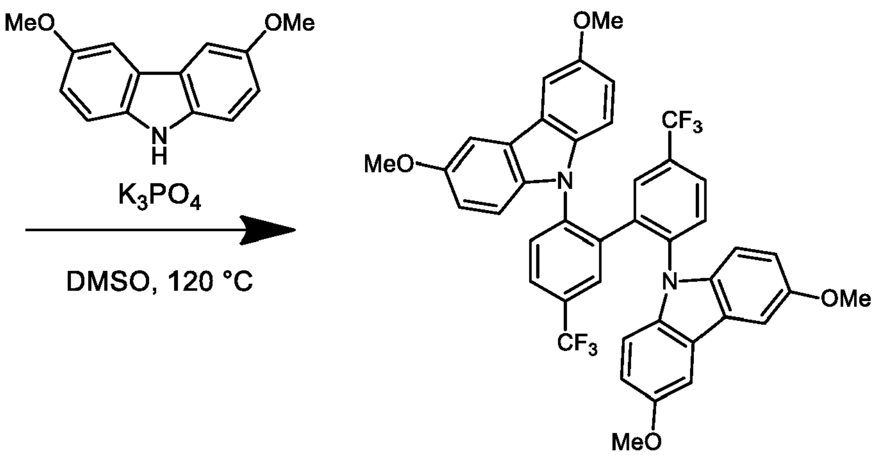


**10**

Stufe 1:

3-Brom-4-flruorbenzotrifluorid (65.8 mmol), Bis(pinacolato)diboron (32.9 mmol), $Pd_2(dba)_3$ (0.33 mmol), SPhos (1.32 mmol) und tribasisches Kaliumphosphat (197 mmol) werden unter Stickstoff in einem Dioxan/Wasser-Gemisch (300 mL / 20 mL) bei 110 °C für 16 h gerührt. Anschließend werden die unlöslichen Bestandteile der Reaktionsmischung abfiltriert und mit Dioxan nachgewaschen. Das Lösemittel des Filtrats wird entfernt und der erhaltene Rückstand in Dichlormethan gelöst und über wenig Kiesegel filtriert. Das Produkt wird als gelber Feststoff erhalten. $^1$H NMR (500 MHz, Chloroform-*d*) $\delta$ = 7.72 - 7.69 (m, 4H), 7.34 - 7.31 (m, 2H) ppm.

Stufe 2:

**10a** (5 mmol), 3,6-Dimethoxycarbazol (12 mmol) und tribasisches Kaliumphosphat (20 mmol) werden unter Stickstoff in DMSO (20 mL) suspendiert und bei 100 °C gerührt (16 h). Anschließend wird die Reaktionsmischung in 150 mL Wasser gegeben und mit Dichlormethan (2 x 100 mL) extrahiert. Die vereinigten organischen Phasen werden mit ges. Natriumchlorid-Lösung gewaschen (2 x 150 mL), getrocknet über Magnesiumsulfat und das Lösemittel anschließend entfernt. Das Rohprodukt wurde in Dichlormethan aufgenommen und über wenig Kieselgel filtriert (Eluent: Dichlormethan). Nach entfernen des Lösemittels wurde schließlich durch Umkristallisation aus einer Mischung von Ethanol und Chloroform gereinigt. Das Produkt **10** wurde als weißer Feststoff erhalten.

$^1$H NMR (500 MHz, Chloroform-d) $\delta$ 8.15 (d, J = 2.1 Hz, 2H), 7.59 (dd, J = 8.3, 2.1 Hz, 2H), 7.23 (d, J = 8.3 Hz, 3H), 6.94 (br s, 2H), 6.79-6.48 (br m, 4H), 6.36 (br s, 2H), 5.68 (br s, 2H), 3.86 (s, 12H).

Die Filmemission von **10** (10 % in PMMA) ist Figur 13 zu entnehmen. Das Emissionsmaximum liegt bei 461 nm. Die Photolumineszenzquantenausbeute (PLQY) beträgt 47%.

**Beispiel 11**

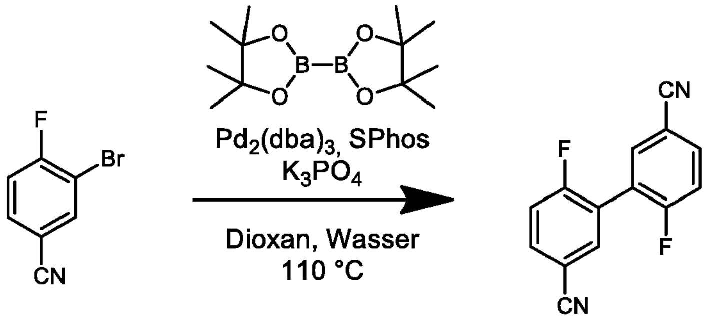


**11a**

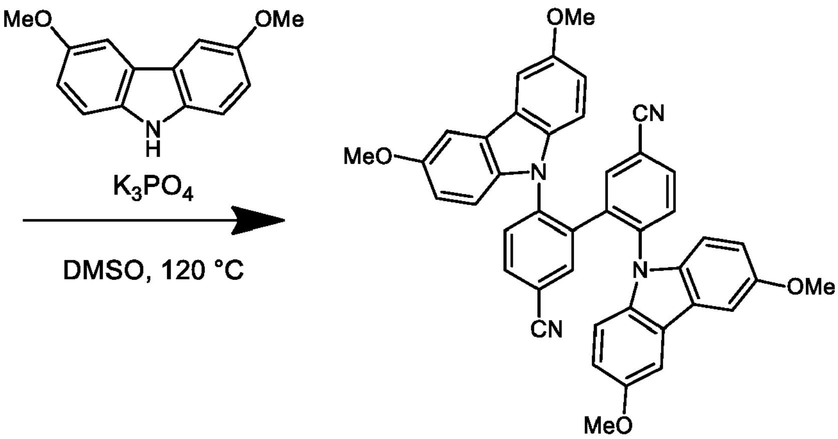


**11**

Stufe 1:

3-Brom-4-fluorbenzonitril (125 mmol), Bis(pinacolato)diboron (62.5 mmol), $Pd_2(dba)_3$ (0.63 mmol), SPhos (2.50 mmol) und tribasisches Kaliumphosphat (375 mmol) werden unter Stickstoff in einem Dioxan/Wasser-Gemisch (350 mL / 20 mL) bei 110 °C für 16 h gerührt. Anschließend werden die unlöslichen Bestandteile der Reaktionsmischung abfiltriert und mit Dioxan nachgewaschen. Das Lösemittel des Filtrats wird entfernt und der erhaltene Rückstand in Tetrahydrofuran gelöst und über wenig Kiesegel filtriert. Das Produkt wird als gelber Feststoff erhalten. $^1$H NMR (500 MHz, Chloroform-*d*) $\delta$ = 7.79 - 7.76 (m, 2H), 7.74 - 7.73 (m, 2H), 7.36 - 7.33 (m, 2H) ppm.

Stufe 2:

**11a** (8.33 mmol), 3,6-Dimethoxycarbazol (19.2 mmol) und tribasisches Kaliumphosphat (33.3 mmol) werden unter Stickstoff in DMSO (30 mL) suspendiert und bei 110 °C gerührt (16 h). Anschließend wird die Reaktionsmischung in 400 mL ges. Natriumchlorid-Lösung gegeben und mit Dichlormethan (3 x 150 mL) extrahiert. Die vereinigten organischen Phasen werden mit ges. Natriumchlorid-Lösung gewaschen (2 x 150 mL), getrocknet über Magnesiumsulfat und das Lösemittel anschließend entfernt. Das Rohprodukt wurde schließlich durch Umkristallisation aus Toluol gereinigt. Das Produkt **11** wurde als gelber Feststoff erhalten.

$^1$H NMR (500 MHz, Chloroform-*d*) $\delta$ = 8.19 (d, J = 1.9 Hz, 2H), 7.62 (dd, J = 8.3, 2.0 Hz, 2H), 7.24 (d, J = 8.3 Hz, 2H), 7.21 (br s, 2H), 6.87 (br s, 2H), 6.72 (br s, 2H), 6.49 (br s, 2H), 6.38 (br s, 2H), 5.65 (br s, 2H), 3.94 - 3.75 (m, 12H).

Die Filmemission von **11** (10 % in PMMA) ist Figur 14 zu entnehmen. Das Emissionsmaximum liegt bei 478 nm. Die Photolumineszenzquantenausbeute (PLQY) beträgt 75%. Die Emissionslebensdauer beträgt 25 μs.

**Beispiel 12**

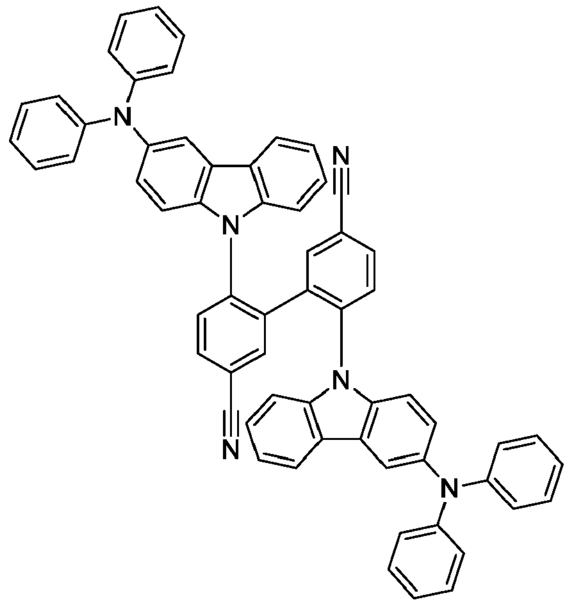

**12**

Dünschichtchromatografie: $R_f$ = 0.5 (Cyclohexan/Ethylacetat 5:1)

Die Filmemission von **12** (10 % in PMMA) ist Figur 15 zu entnehmen. Das Emissionsmaximum liegt bei 495 nm. Die Photolumineszenzquantenausbeute (PLQY) beträgt 64%. Die Emissionslebensdauer beträgt 13 μs.

**Beispiel 13**

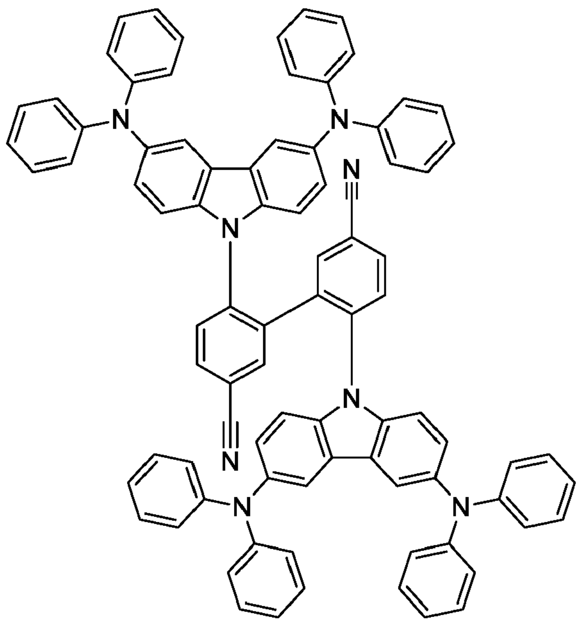

**13**

Dünschichtchromatografie: $R_f$ = 0.5 (Cyclohexan/Ethylacetat 5:1)

Die Filmemission von **13** (10 % in PMMA) ist Figur 16 zu entnehmen. Das Emissionsmaximum liegt bei 507

nm. Die Photolumineszenzquantenausbeute (PLQY) beträgt 66%. Die Emissionslebensdauer beträgt 10 μs.

**Beispiel 14**

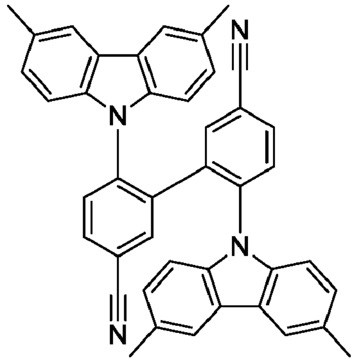

**14**

Dünschichtchromatografie: $R_f$ = 0.5 (Cyclohexan/Ethylacetat 5:1)

Die Filmemission von **14** (10 % in PMMA) ist Figur 17 zu entnehmen. Das Emissionsmaximum liegt bei 450 nm. Die Photolumineszenzquantenausbeute (PLQY) beträgt 62 %.

**Beispiel 15**

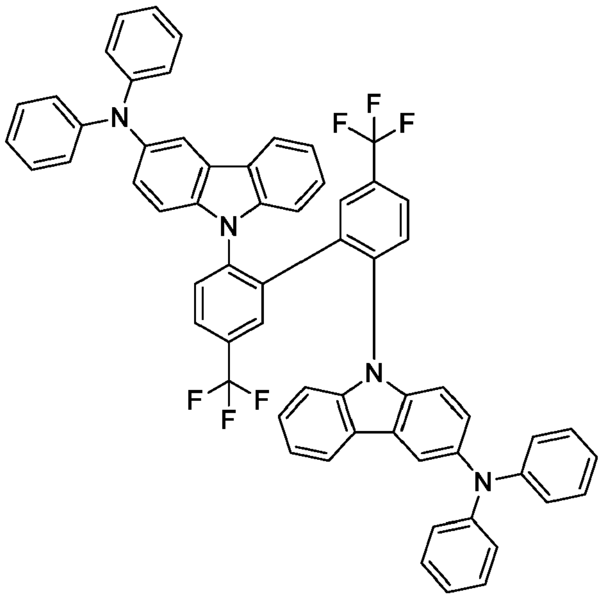

**15**

Dünschichtchromatografie: $R_f$ = 0.6 (Cyclohexan/Ethylacetat 10:1)

Die Filmemission von **15** (10 % in PMMA) ist Figur 18 zu entnehmen. Das Emissionsmaximum liegt bei 472 nm. Die Photolumineszenzquantenausbeute (PLQY) beträgt 32 %.

**Beispiel 16**

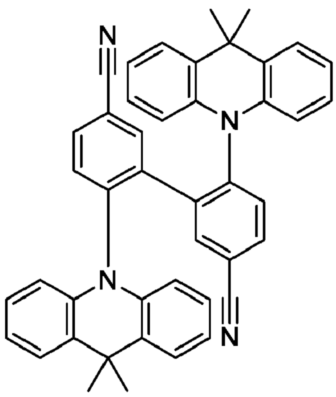

**16**

$^1H$ NMR (500 MHz, Chloroform-d) δ 7.79 (dd, J = 8.2, 2.0 Hz, 2H), 7.63 (d, J = 2.0 Hz, 2H), 7.48 - 7.32 (m, 6H), 7.20-5.30 (br m, J = 510.3 Hz, 12H), 1.87 (s, 6H), 0.80 (s, 6H).

Die Filmemission von **16** (10 % in PMMA) ist Figur 19 zu entnehmen. Das Emissionsmaximum liegt bei 471 nm. Die Photolumineszenzquantenausbeute (PLQY) beträgt 65%. Die Emissionslebensdauer beträgt 21 μs.

**Beispiel 17 - OLED-Bauteil A**

Molekül **1** wurde in einem OLED-Bauteil mit folgendem Aufbau getestet: ITO/m-MTDATA/HAT-CN/tris-Pcz/**1**:DPEPO (20%)/TSPO1/BPyTP2/Liq/Al

Tabelle 1: Bauteildaten für das Bauteil A.

| | |
|---|---|
| Leistungseffizienz | 7.5 lm/W |
| Stromeffizienz | 9.7 cd/A |
| CIE | CIEx: 0.16<br>CIEy: 0.176<br>@ 13 V |
| Externe Quantenausbeute (EQE) | 6.8% |

Die Stromdichte und Leuchtdichte über der Spannung sind in Figur 19 dargestellt. Die Leistungseffizienz und Stromeffizienz über der Spannung sind in Figur 20 dargestellt. Die externe Quanteneffizienz über der Stromdichte ist in Figur 21 dargestellt. Das Elektrolumineszenzspektrum betrieben bei 10 V der OLED ist in Figur 22 dargestellt.

**Beispiel 18 - OLED-Bauteil B**

Molekül **1** wurde in einem OLED-Bauteil mit folgendem Aufbau getestet: ITO/m-MTDATA/α-NPD/TCTA/Cz-Si/**1**(10%):DPEPO/DPEPO/TPBi/Liq/Al

Tabelle 2: Bauteildaten für das Bauteil B.

| | |
|---|---|
| Leistungseffizienz | 7.7 lm/W |
| Stromeffizienz | 13.8 cd/A |
| CIE | CIEx: 0.16<br>CIEy: 0.17<br>@ 14 V |
| Externe Quantenausbeute (EQE) | 10.1% |

Weitere Beispiele organischer Moleküle mit einer Struktur gemäß Formel I:

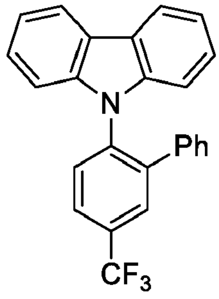
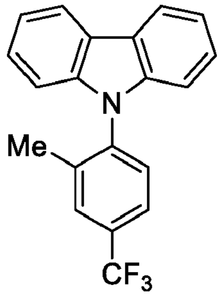
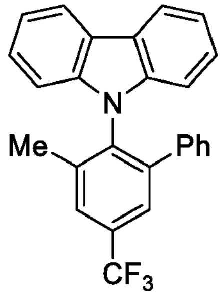
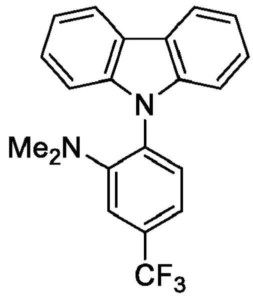
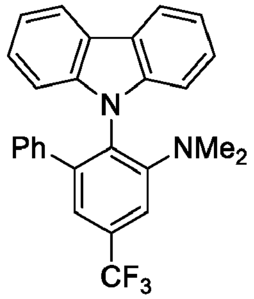
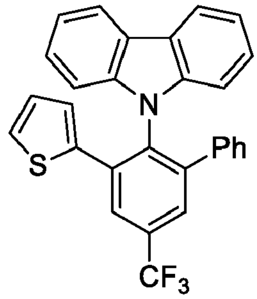
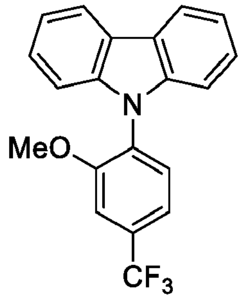
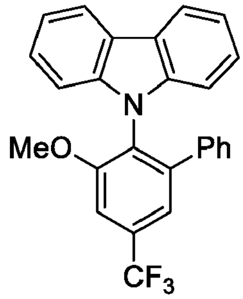
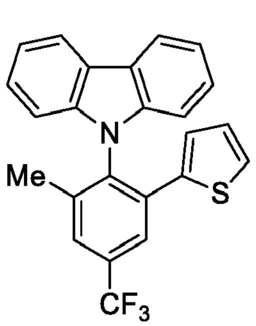
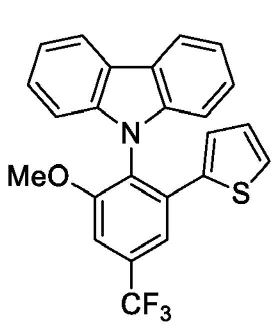
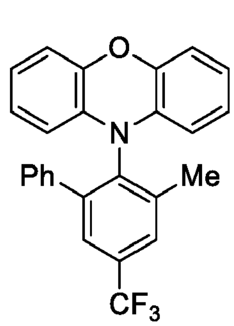
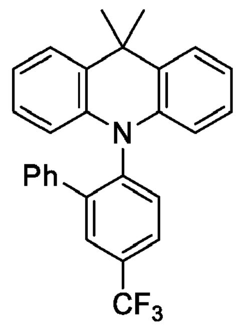
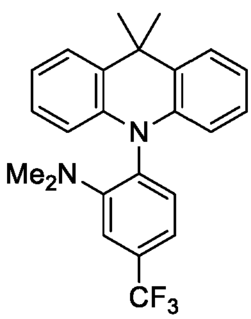
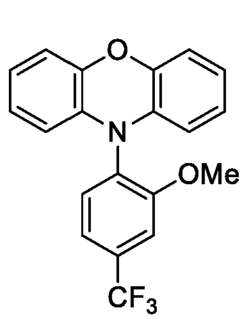
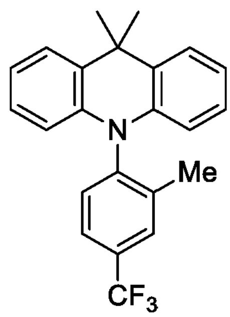
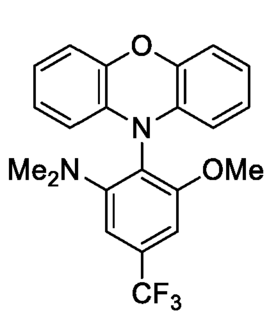
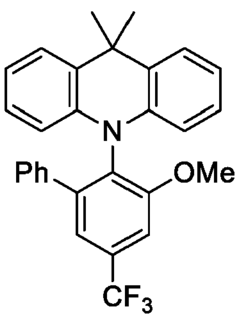
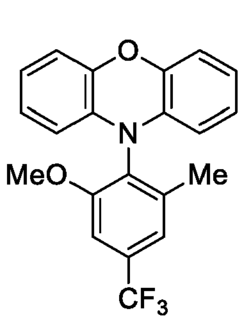
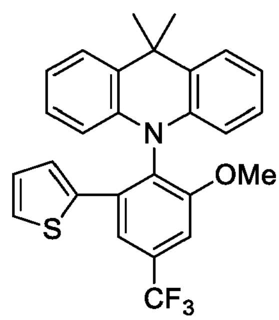
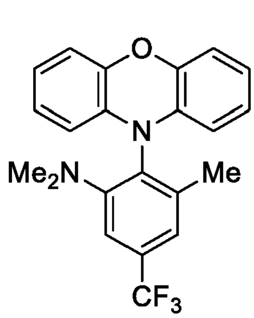

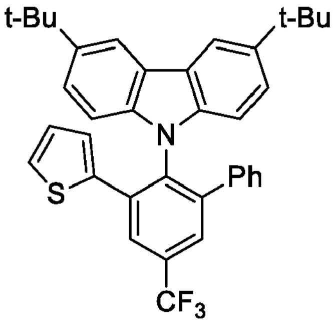
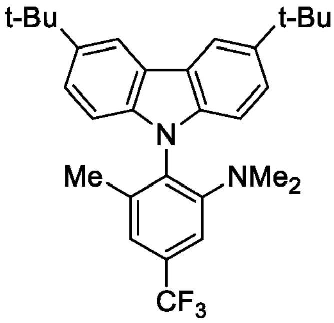
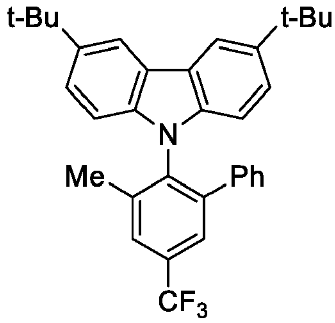

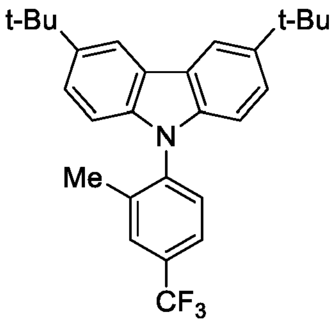
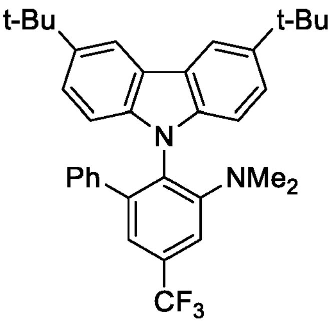
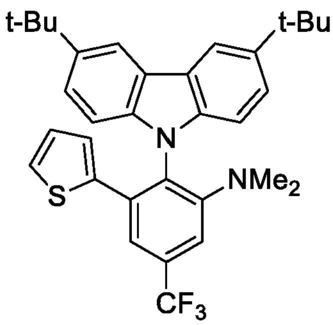

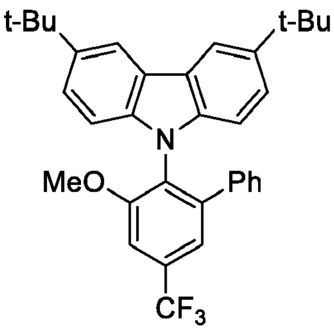
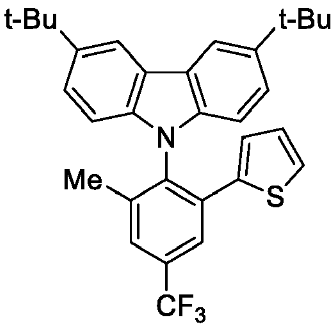
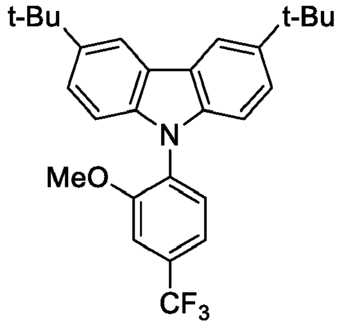

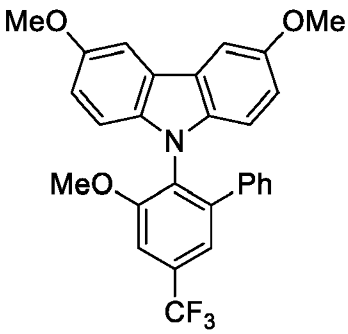
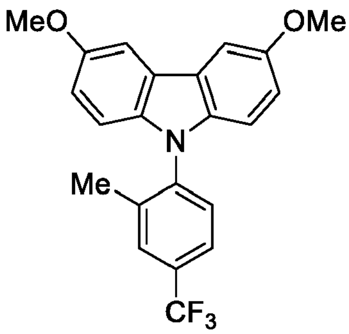
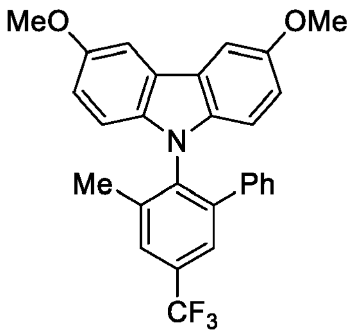

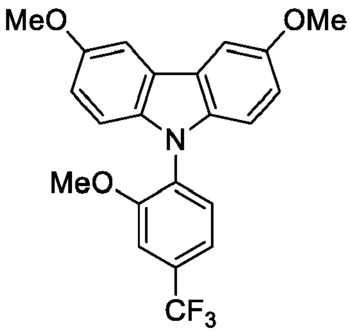

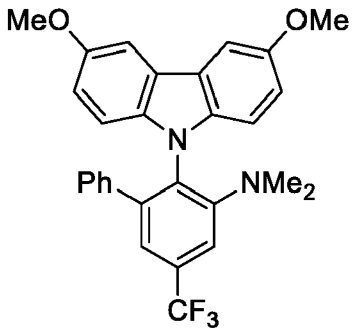

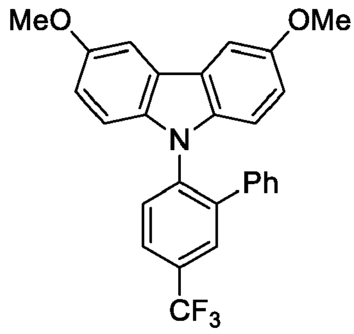

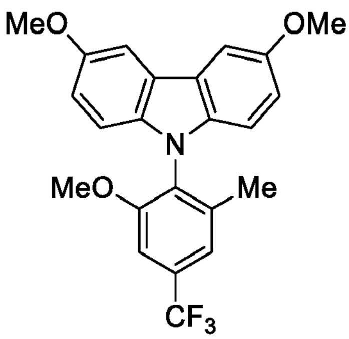

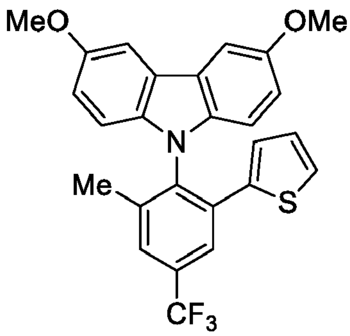

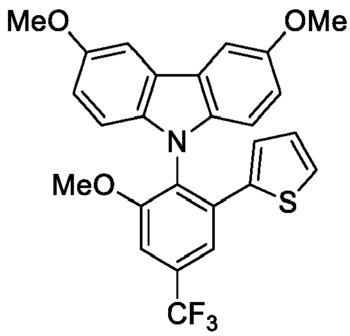

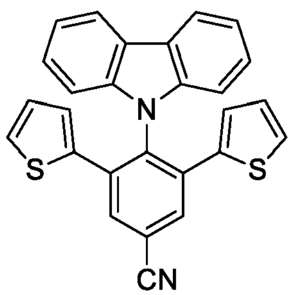

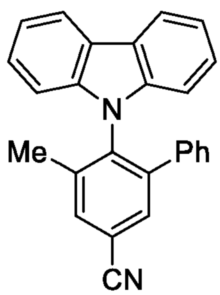

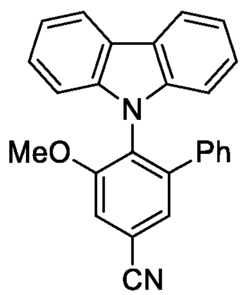

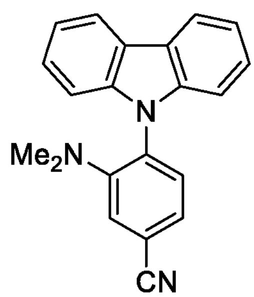

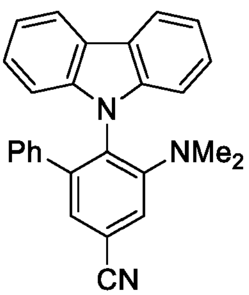

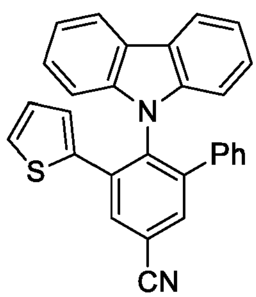

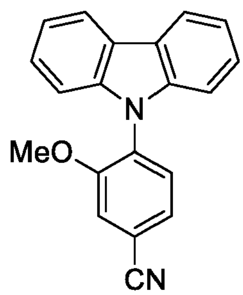

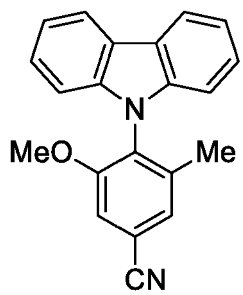

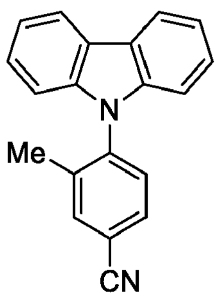

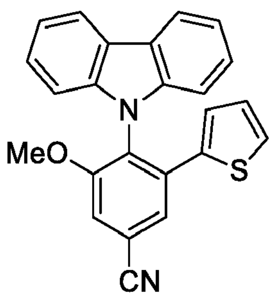

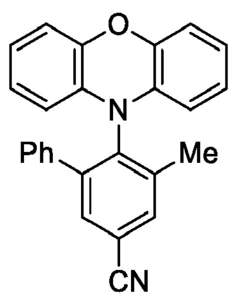

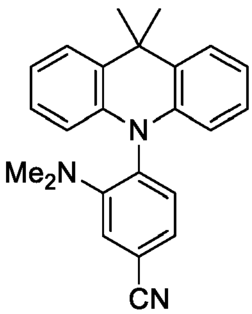
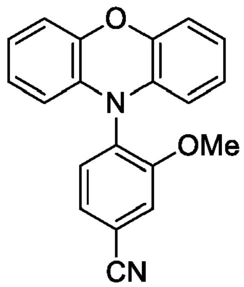
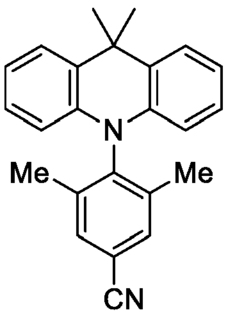
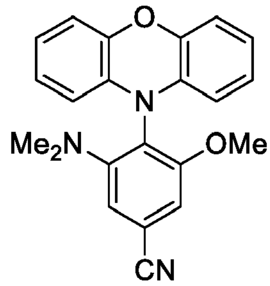
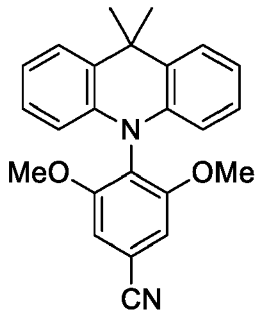
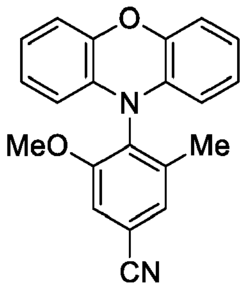
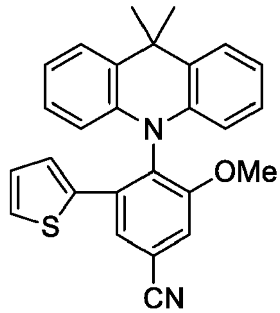
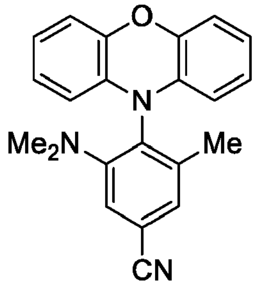
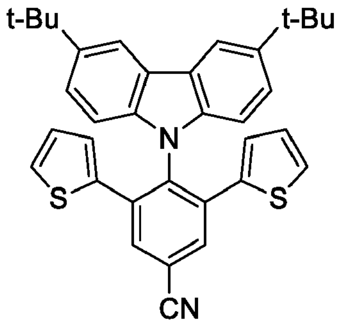
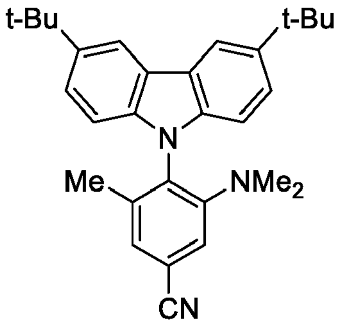
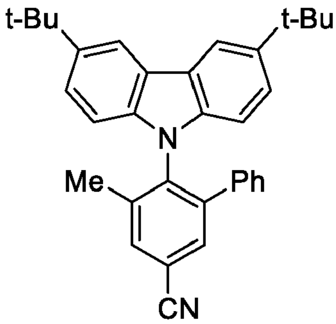
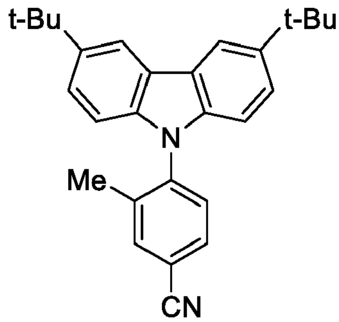
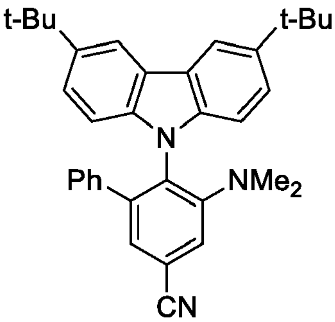
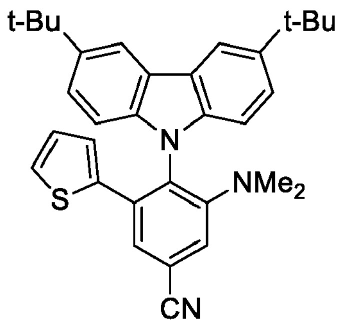

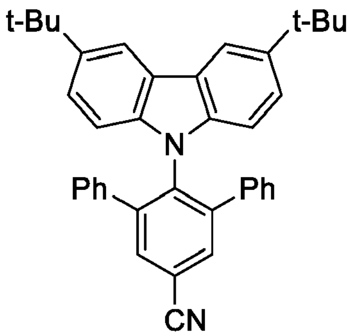
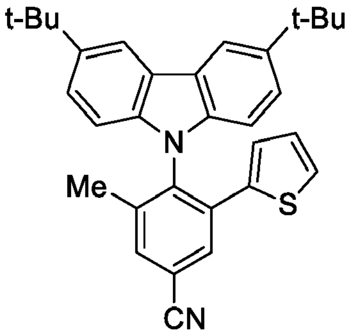
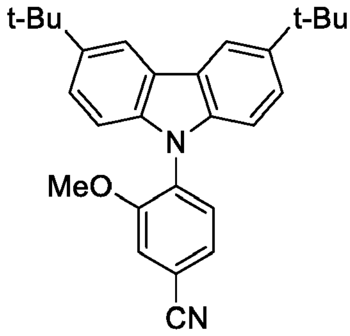

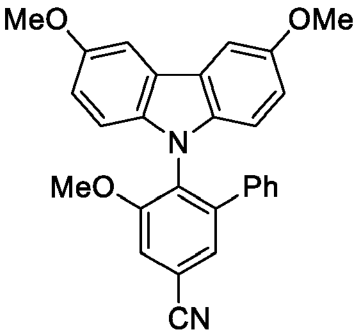
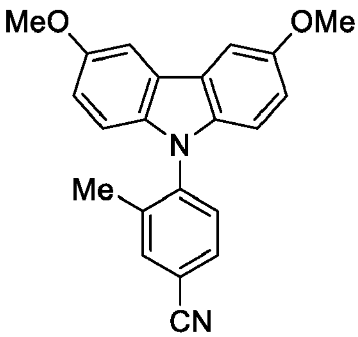
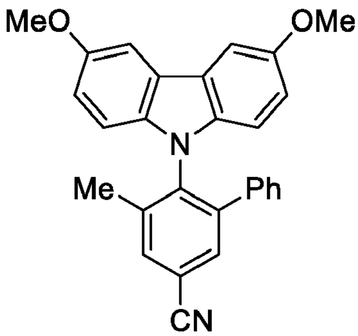

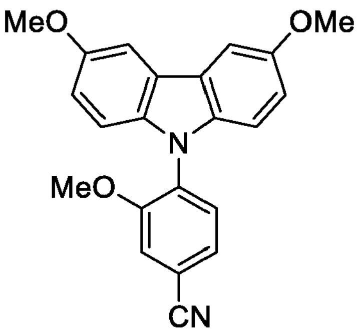
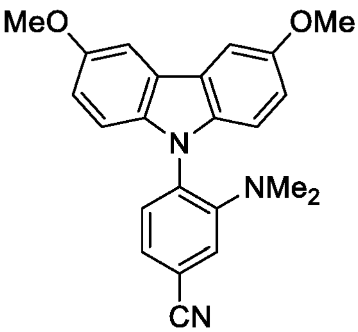
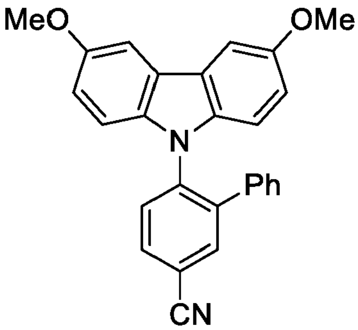

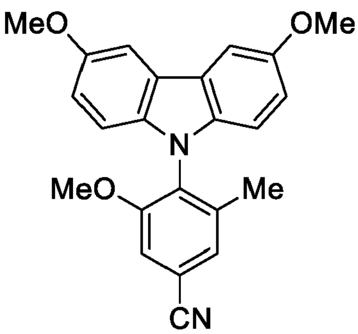
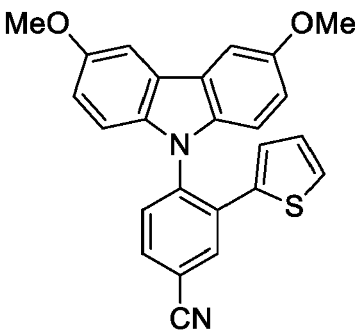
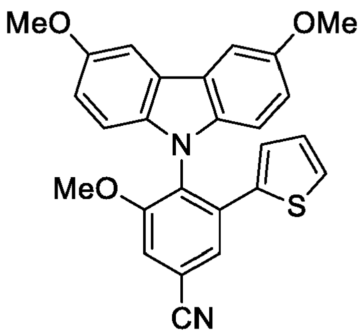

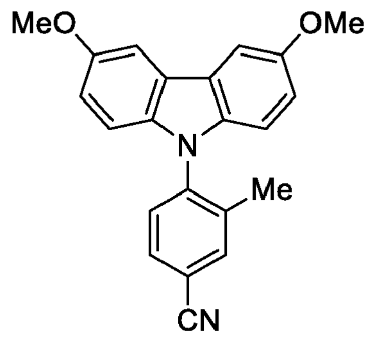

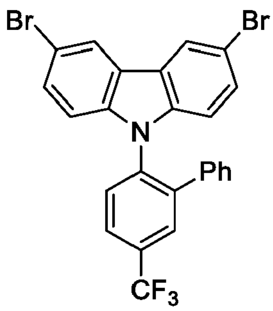

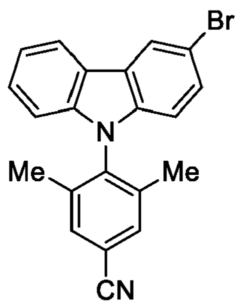

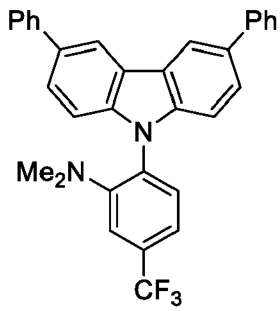

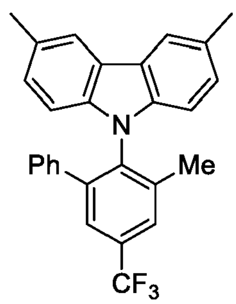

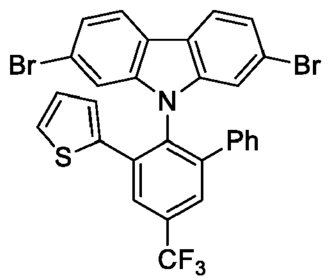

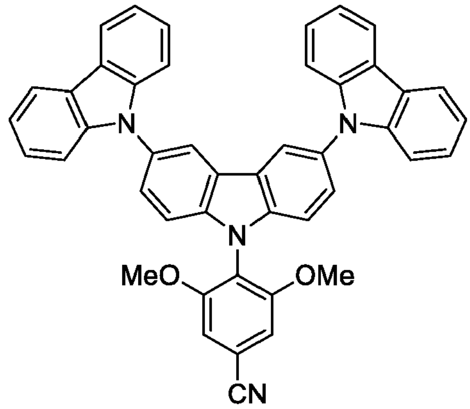

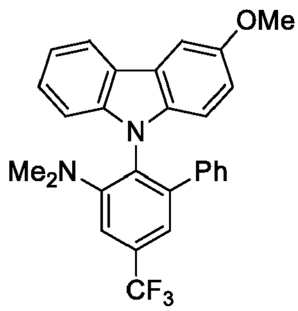

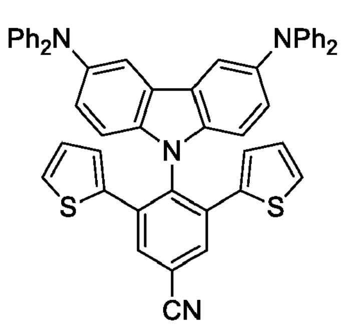

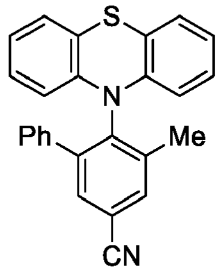

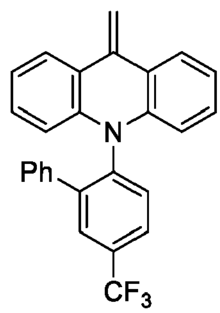

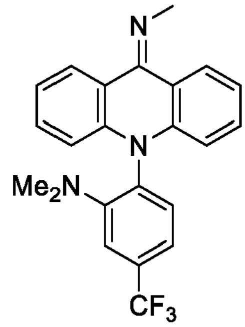

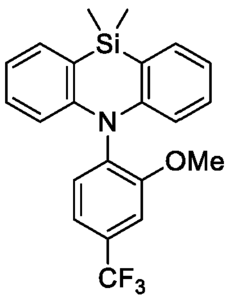
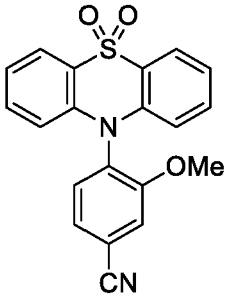
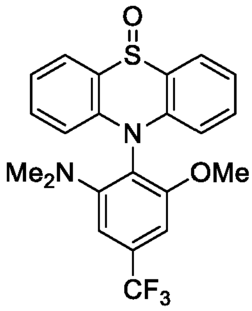
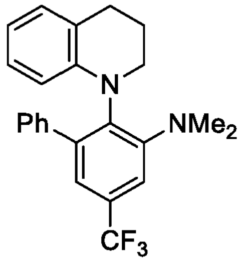

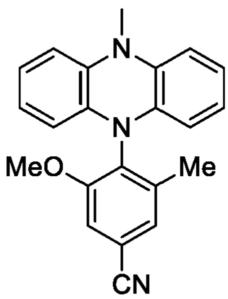
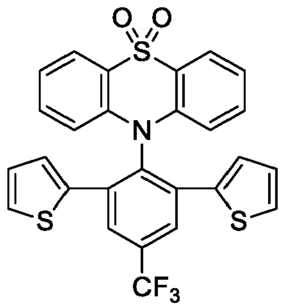
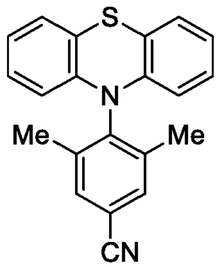

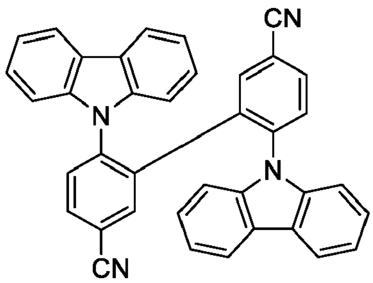
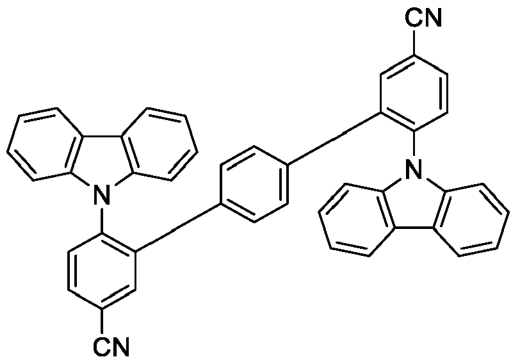

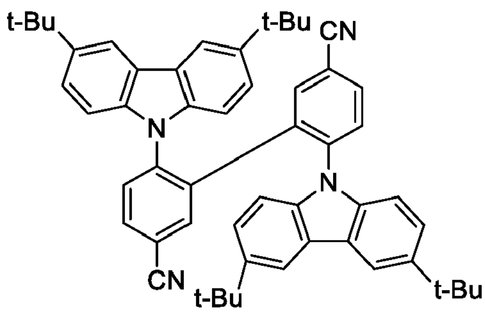
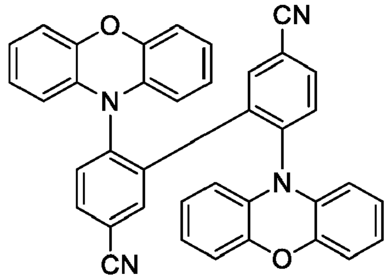

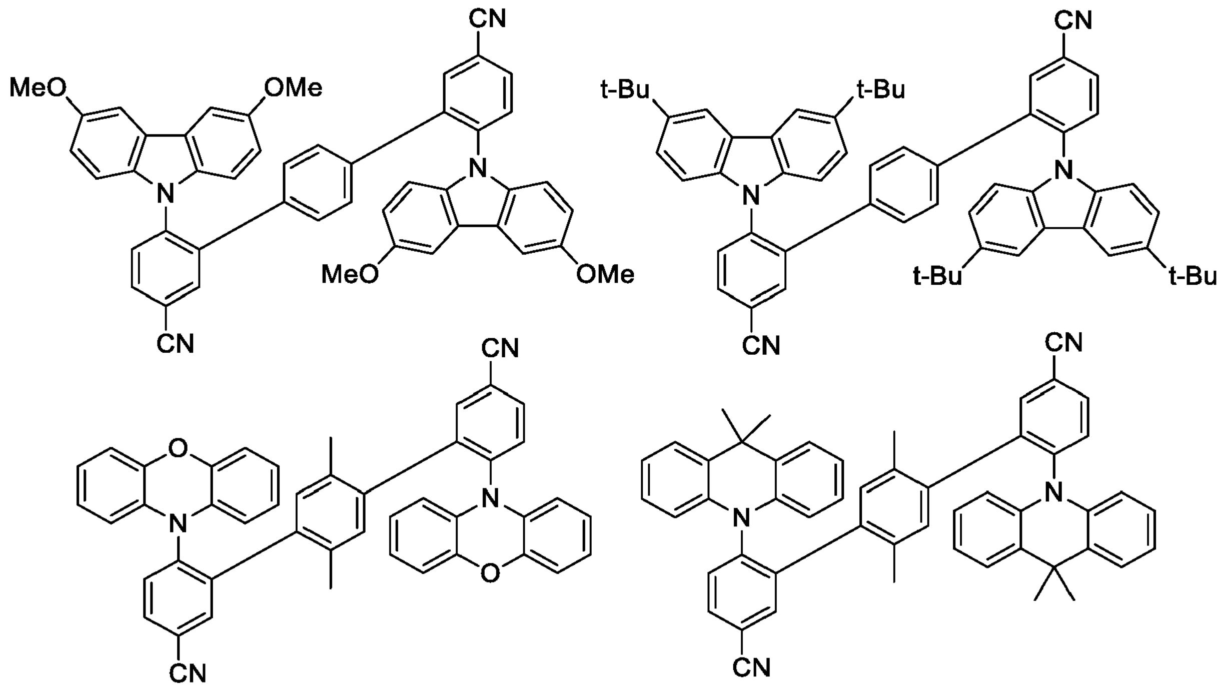

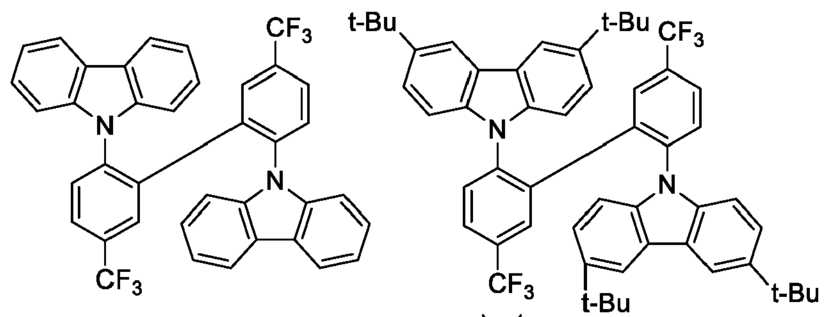

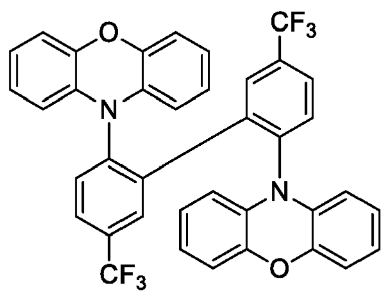

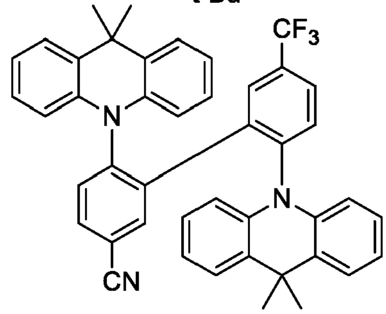

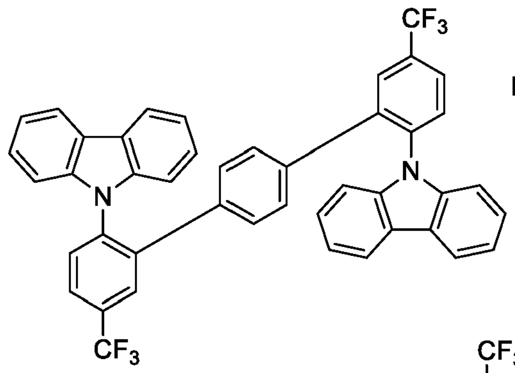

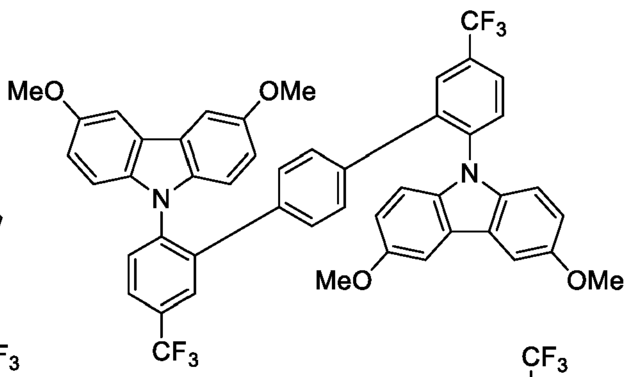

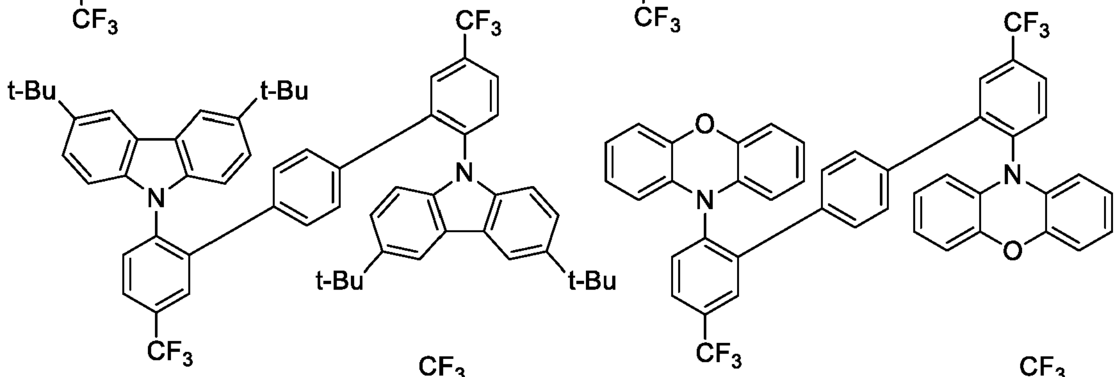

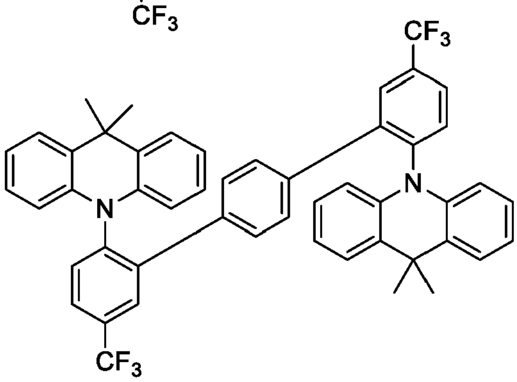

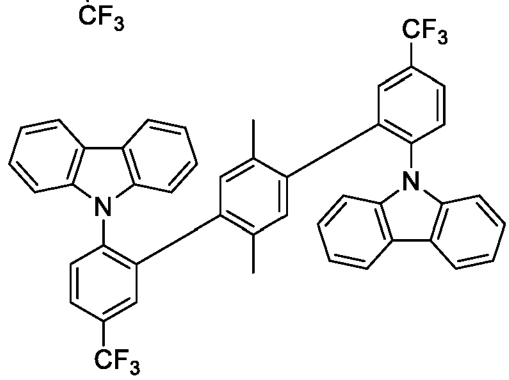

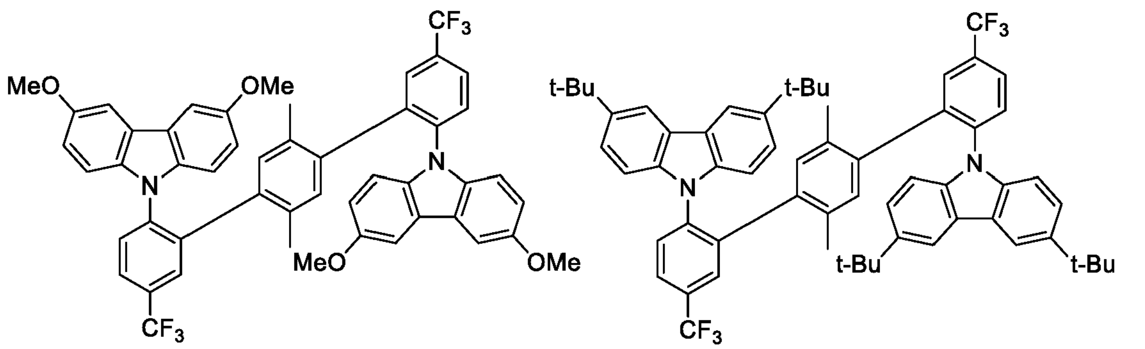

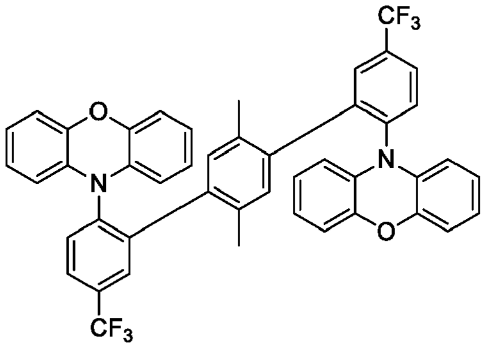

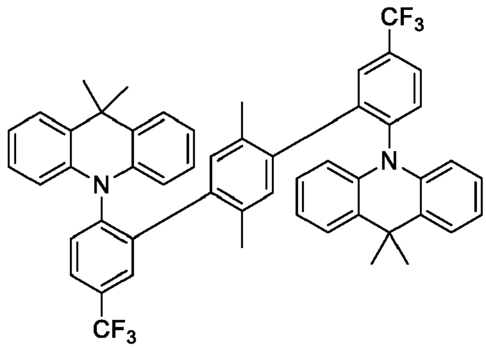

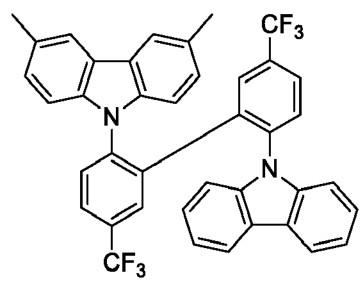

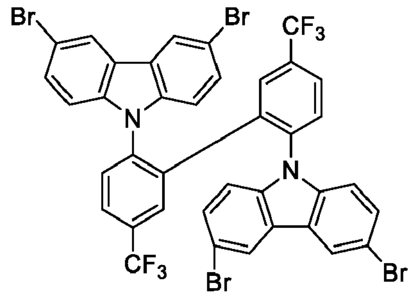

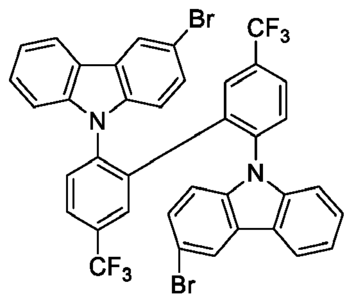

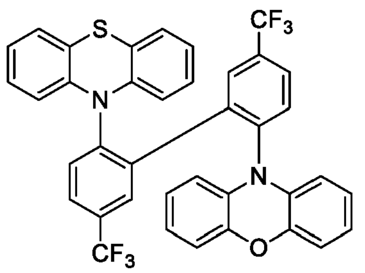

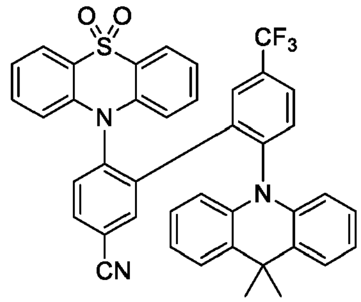

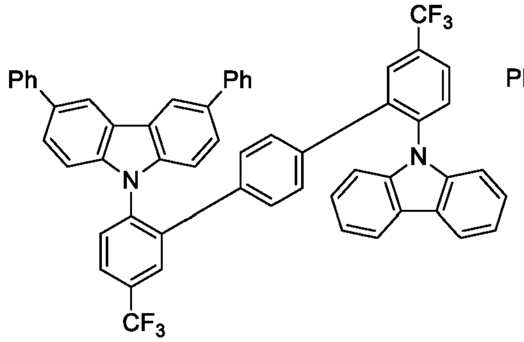

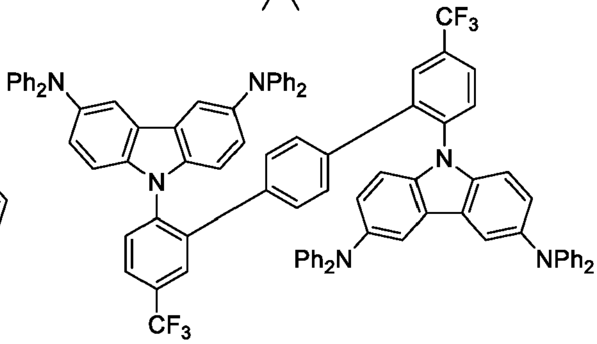

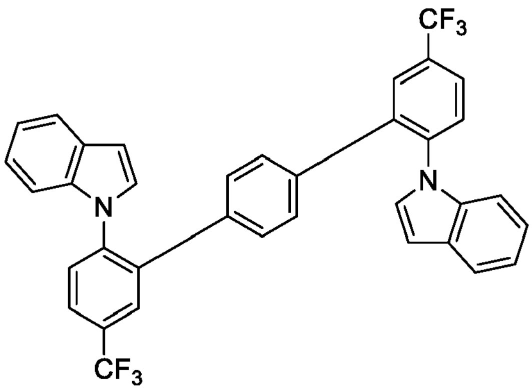

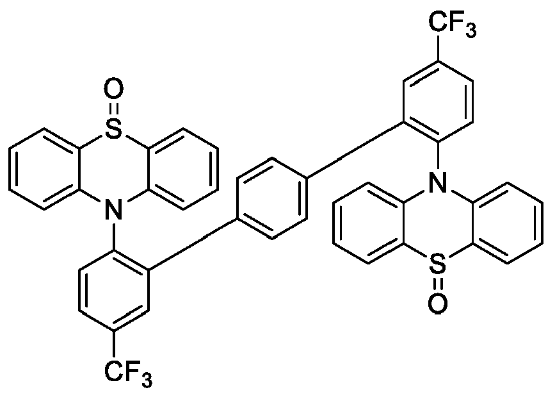

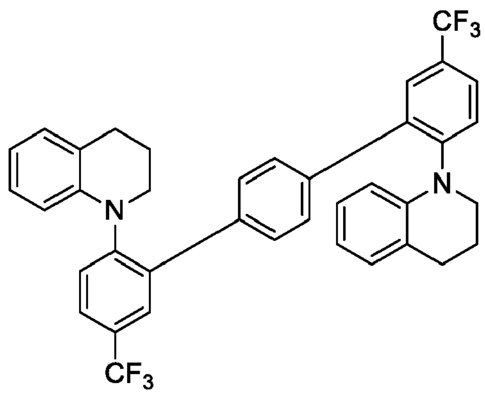

**Figuren**

Figur 1: Absorptionsspektrum von **1** als Lösung in 2-Methyltetrahydrofuran.
Figur 2: Filmemission von **1** (10 % in PMMA).
Figur 3 Berechnete Grenzorbitale des Grundzustands von **1**.
Figur 4: Absorptionsspektrum von **2** als Lösung in 2-Methyltetrahydrofuran.
Figur 5: Filmemission von **2** (10 % in PMMA).
Figur 6: Filmemission von **3** (10 % in PMMA).
Figur 7: Filmemission von **4** (10 % in PMMA).
Figur 8: Filmemission von **5** (10 % in PMMA).
Figur 9: Filmemission von **6** (10 % in PMMA).
Figur 10: Filmemission von **7** (10 % in PMMA).
Figur 11: Filmemission von **8** (10 % in PMMA).Figur 12: Filmemission von **9** (10 % in PMMA).
Figur 13: Filmemission von **10** (10 % in PMMA).
Figur 14: Filmemission von **11** (10 % in PMMA).
Figur 15: Filmemission von **12** (10 % in PMMA).
Figur 16: Filmemission von **13** (10 % in PMMA).
Figur 17: Filmemission von **14** (10 % in PMMA).
Figur 18: Filmemission von **15** (10 % in PMMA).
Figur 19: Stromdichte und Leuchtdichte des OLED-Bauteils ITO/m-MTDATA/HAT-CN/tris-Pcz/ **1**:DPEPO (20 %)/TSPO1/BPyTP2/Liq/Al.
Figur 20: Leistungseffizienz und Stromeffizienz über der Spannung des OLED-Bauteils ITO/m-MTDATA/HAT-CN/tris-Pcz/ **1**:DPEPO (20 %)/TSPO1/BPyTP2/Liq/Al.
Figur 21: Externe Quanteneffizienz über der Stromdichte des OLED-Bauteils ITO/m-MTDATA/HAT-CN/tris-Pcz/ **1**:DPEPO (20 %)/TSPO1/BPyTP2/Liq/Al
Figur 22: Elektrolumineszenzspektrum betrieben bei 10 V des OLED-Bauteils ITO/m-MTDATA/HAT-CN/tris-Pcz/ **1**:DPEPO (20 %)/TSPO1/BPyTP2/Liq/Al.

**Patentansprüche**

1. Organisches Molekül, aufweisend eine Struktur der Formel I

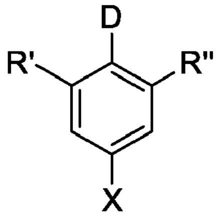

Formel I

oder eine Verbindung enthaltend genau zwei Einheiten gemäß Formel I, die über eine Einfachbindung oder eine Brücke Y miteinander verknüpft sind,
mit

Y = eine divalente oder trivalente Gruppe;
X = eine elektronenziehende Einheit, bevorzugt CN oder $CF_3$;
D = chemische Einheit aufweisend eine Struktur der Formel I-1:

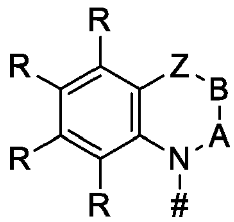

Formel I-1

wobei

# = Anknüpfungspunkt der Einheit gemäß Formel I-1 an die Struktur gemäß Formel I;
A und B = unabhängig voneinander ausgewählt aus der Gruppe bestehend aus $CRR^1$, CR, NR, N, wobei zwischen A und B eine Einfach- oder Doppelbindung vorliegt und zwischen B und Z eine Einfach- oder Doppelbindung vorliegt;
Z = eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination dieser, $-CRR^1$, $-C=CRR^1$, -C=NR, -NR-, -O-, $-SiRR^1-$, -S-,-S(O)-, $-S(O)_2-$, durch O unterbrochene substituierte oder unsubstituierte C1-C9-Alkylen-, C2-C8-Alkenylen-, C2-C8-Alkinylen- oder Arylen-Gruppe, Phenyl- oder substituierte Phenyleinheiten ist;
wobei jedes R und $R^1$ bei jedem Auftreten gleich oder verschieden ist H, Deuterium, Azid ($N_3^-$), F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination dieser

Systeme, oder eine quervernetzbare Einheit QE ist, die durch säurekatalytische, thermische oder UV-Quervernetzungsverfahren in Anwesenheit oder Abwesenheit eines Photoinitiators oder durch Mikrowellenstrahlung quervernetzt werden kann; wobei optional zwei oder mehrere dieser Substituenten R und $R^1$ miteinander ein mono- oder polycyclisches, aliphatisches, aromatisches und/oder benzoannelliertes Ringsystem bilden;

$R^2$ bei jedem Auftreten gleich oder verschieden ist H, Deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; wobei optional zwei oder mehrere dieser Substituenten $R^2$ miteinander ein mono- oder polycyclisches, aliphatisches, aromatisches und/oder benzoannelliertes Ringsystem bilden;

$R^3$ bei jedem Auftreten gleich oder verschieden ist H, Deuterium, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können;

wobei optional zwei oder mehrere Substituenten $R^3$ miteinander ein mono- oder polycyclisches, aliphatisches Ringsystem bilden;

R' = Anknüpfungsposition für die zweite Einheit der Formel I oder ausgewählt aus der Gruppe bestehend aus Y, H, $N(R^4)_2$, $OR^4$, eine lineare Alkyl- oder Alkoxygruppe mit 1 bis 40 C-Atomen oder eine verzweigte oder cyclische Alkyl- oder Alkoxygruppe mit 3 bis 40 C-Atomen, die jeweils mit einem oder mehreren Resten $R^4$ substituiert sein kann, oder ein aromatisches oder heteroaromatisches Ringsystem mit 5 bis 60 aromatischen Ringatomen, das jeweils durch einen oder mehrere Reste $R^4$ substituiert sein kann;

R" = Anknüpfungsposition für die zweite Einheit der Formel I oder ausgewählt aus der Gruppe bestehend aus Y, $N(R^4)_2$, $OR^4$, eine lineare Alkyl- oder Alkoxygruppe mit 1 bis 40 C-Atomen oder eine verzweigte oder cyclische Alkyl- oder Alkoxygruppe mit 3 bis 40 C-Atomen, die jeweils mit einem oder mehreren Resten $R^4$ substituiert sein kann, oder ein aromatisches oder heteroaromatisches Ringsystem mit 5 bis 60 aromatischen Ringatomen, das jeweils durch einen oder mehrere Reste $R^4$ substituiert sein kann;

mit der Maßgabe, dass wenn R' gleich Y ist, R" ungleich Y ist und wenn R" gleich Y ist, R' ungleich Y ist;

und mit der Maßgabe, dass wenn R' gleich der Anknüpfungsposition für die zweite Einheit der Formel I ist, R" ungleich der Anknüpfungsposition für die zweite Einheit der Formel I ist und wenn R" gleich der Anknüpfungsposition für die zweite Einheit der Formel I ist, R' ungleich der Anknüpfungsposition für die zweite Einheit der Formel I ist;

$R^4$ bei jedem Auftreten gleich oder verschieden ist H, Deuterium, $N(R^5)_2$, $Si(R^5)_3$, eine lineare Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^5$ substituiert sein kann, oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann, oder eine Kombination dieser Systeme; wobei optional zwei oder mehrere dieser Substituenten $R^5$ auch miteinander ein mono- oder polycyclisches, aliphatisches, aromatisches und/oder benzoannelliertes Ringsystem bilden;

$R^5$ bei jedem Auftreten gleich oder verschieden ist H, Deuterium, oder ein aliphatischer, aromatischer und/oder heteroaromatischer Kohlenwasserstoffrest mit 1 bis 20 C-Atomen; wobei optional zwei oder mehrere Substituenten $R^5$ auch miteinander ein mono- oder polycyclisches, aliphatisches Ringsystem bilden.

**2.** Organisches Molekül nach Anspruch 1, wobei die Donorgruppe mit elektronenschiebenden Eigenschaften der Formel I-1 eine Struktur der Formel II aufweist:

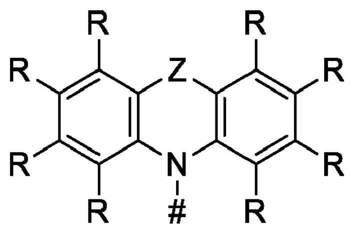

Formel II

wobei für #, Z und R die in Anspruch 1 genannten Definitionen gelten.

**3.** Organisches Molekül nach Anspruch 1, wobei die Donorgruppe mit elektronenschiebenden Eigenschaften der Formel I-1 eine Struktur der Formel III aufweist:

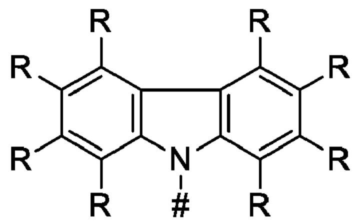

Formel III

wobei für # und R die in Anspruch 1 genannten Definitionen gelten.

**4.** Organisches Molekül nach Anspruch 1 bis 3, wobei die akzeptierende Einheit X der Formel I gleich CN ist.

**5.** Organisches Molekül nach Anspruch 1 bis 3, wobei die akzeptierende Einheit X der Formel I gleich $CF_3$ ist.

**6.** Organisches Molekül nach Anspruch 1 bis 5, wobei der Rest R' der Formel I ein Wasserstoffatom H ist.

**7.** Organisches Molekül nach Anspruch 1 bis 6, wobei die Brücke Y ausgewählt ist aus den Strukturen der Formeln IV bis X

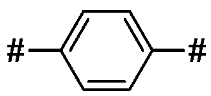

IV

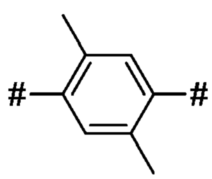

V

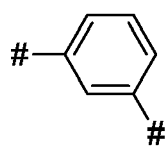

VI

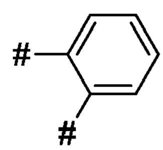

VII

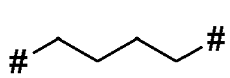

VIII

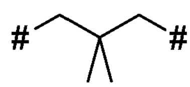

IX

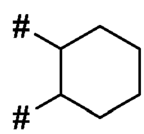

X

mit

# = Anknüpfungspunkt der Brücke Y an die Struktur gemäß Formel I.

**8.** Verfahren zur Herstellung eines Organischen Moleküls nach Anspruch 1 bis 7.

**9.** Verwendung eines organischen Moleküls nach Anspruch 1 bis 7 als lumineszierender Emitter oder als Absorber und/oder als Hostmaterial und/oder als Elektronentransportmaterial und/oder als Lochinjektionsmaterial und/oder als Lochblockiermaterial in einer organischen optoelektronischen Vorrichtung.

**10.** Verwendung nach Anspruch 9, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**11.** Verwendung nach Anspruch 9 oder 10, wobei der Anteil des organischen Moleküls an der Emissionsschicht in einer organischen optoelektronischen Vorrichtung, insbesondere in OLEDs, 1 % bis 99 %, insbesondere 5 % bis 80 % beträgt.

**12.** Organische optoelektronische Vorrichtung, aufweisend ein organisches Molekül nach Anspruch 1 bis 7, insbesondere ausgeformt als eine Vorrichtung ausgewählt aus der Gruppe bestehend ausorganischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**13.** Organische optoelektronische Vorrichtung nach Anspruch 12, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organische Molekül nach Anspruch 1 bis 7 aufweist.

**14.** Verfahren zur Herstellung eines optoelektronischen Bauelements, wobei ein organisches Molekül nach Anspruch 1 bis 7 verwendet wird.

**15.** Verfahren nach Anspruch 14, umfassend die Verarbeitung des organischen Moleküls mittels eines Vakuumverdampfungsverfahrens oder aus einer Lösung.

Figur 1

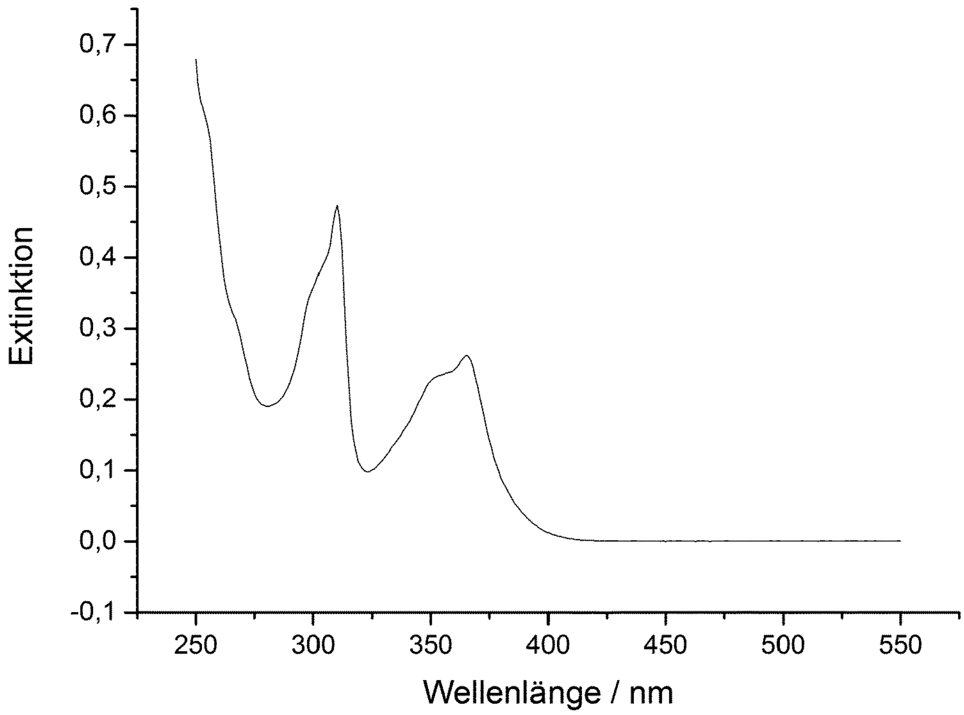


Figur 2

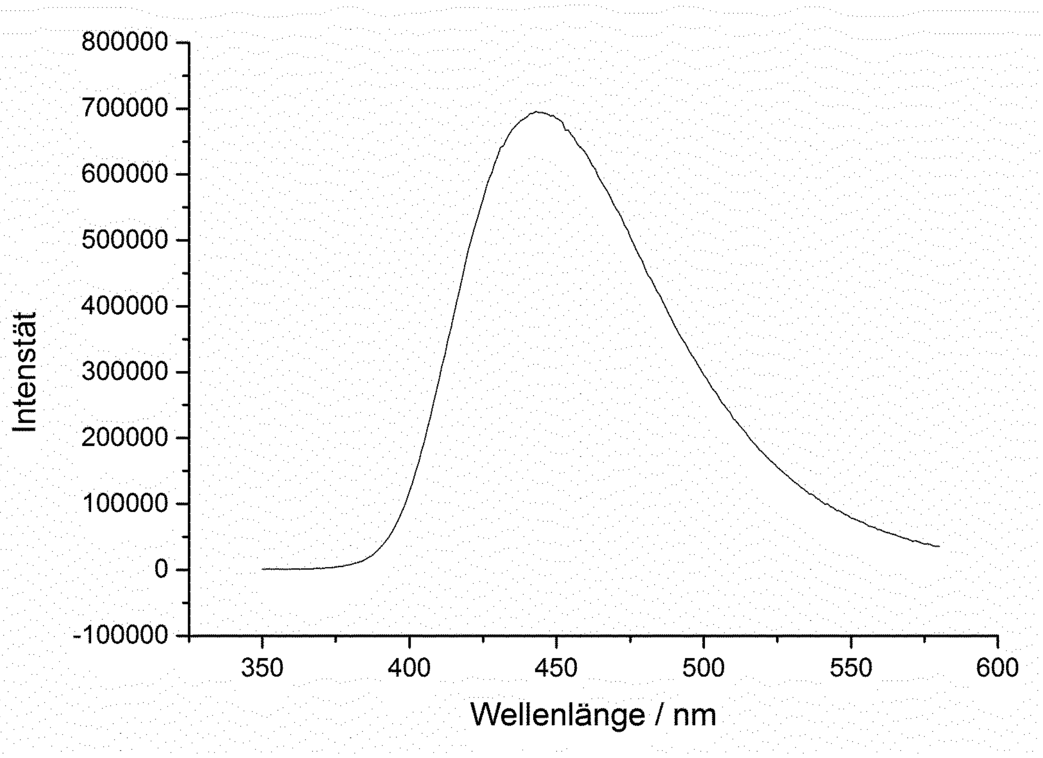

Figur 3

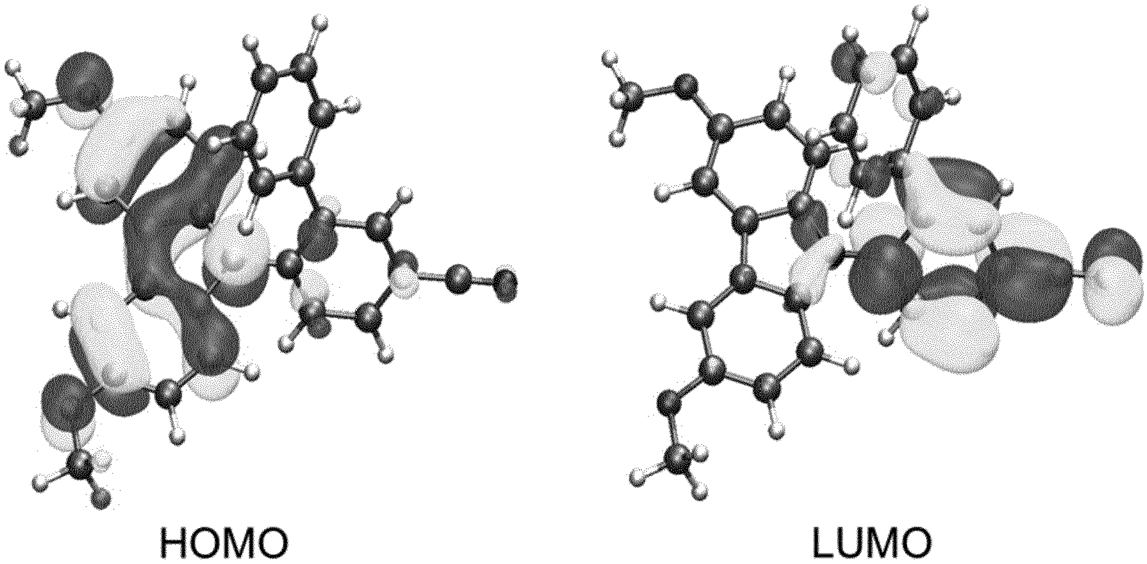


Figur 4

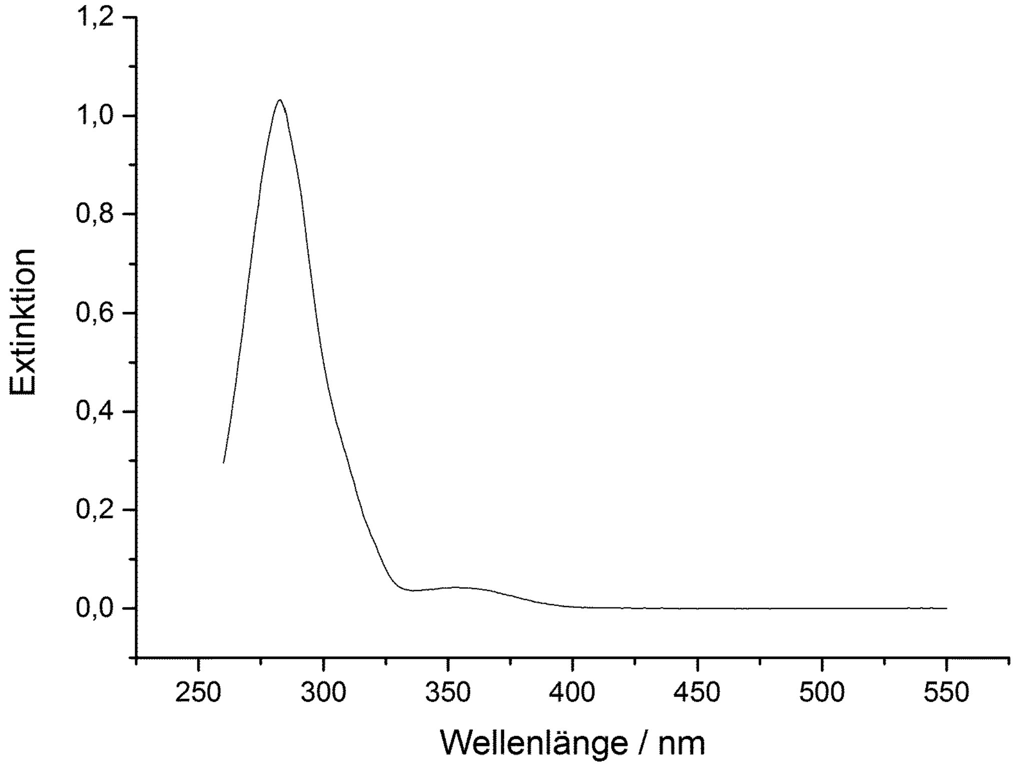

Figur 5

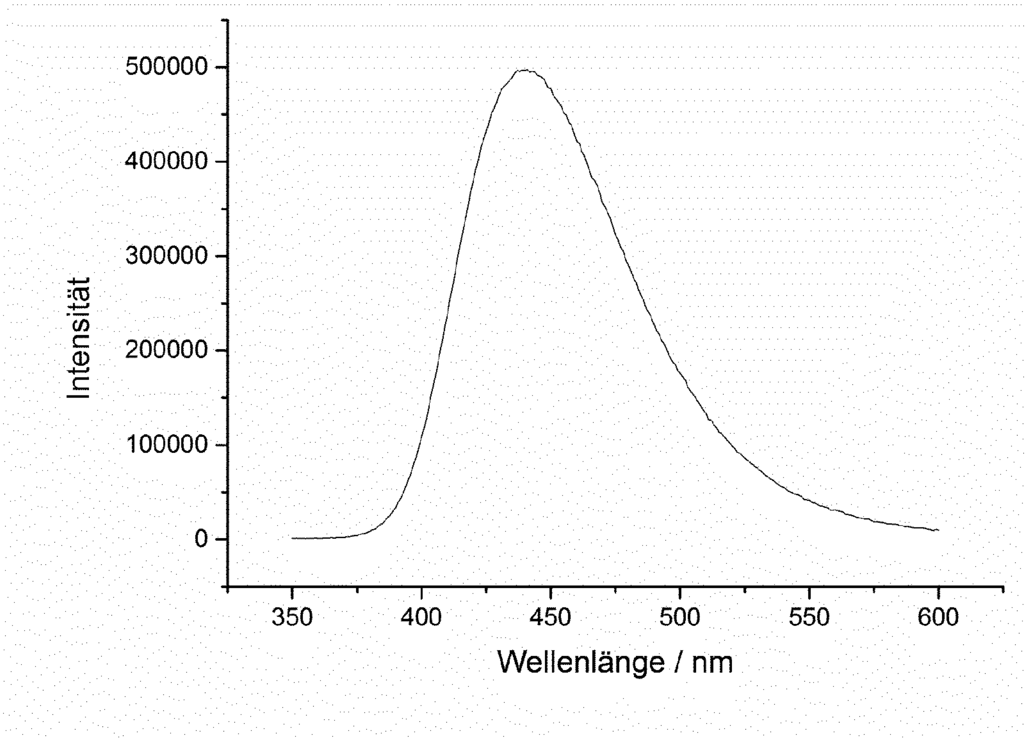


Figur 6

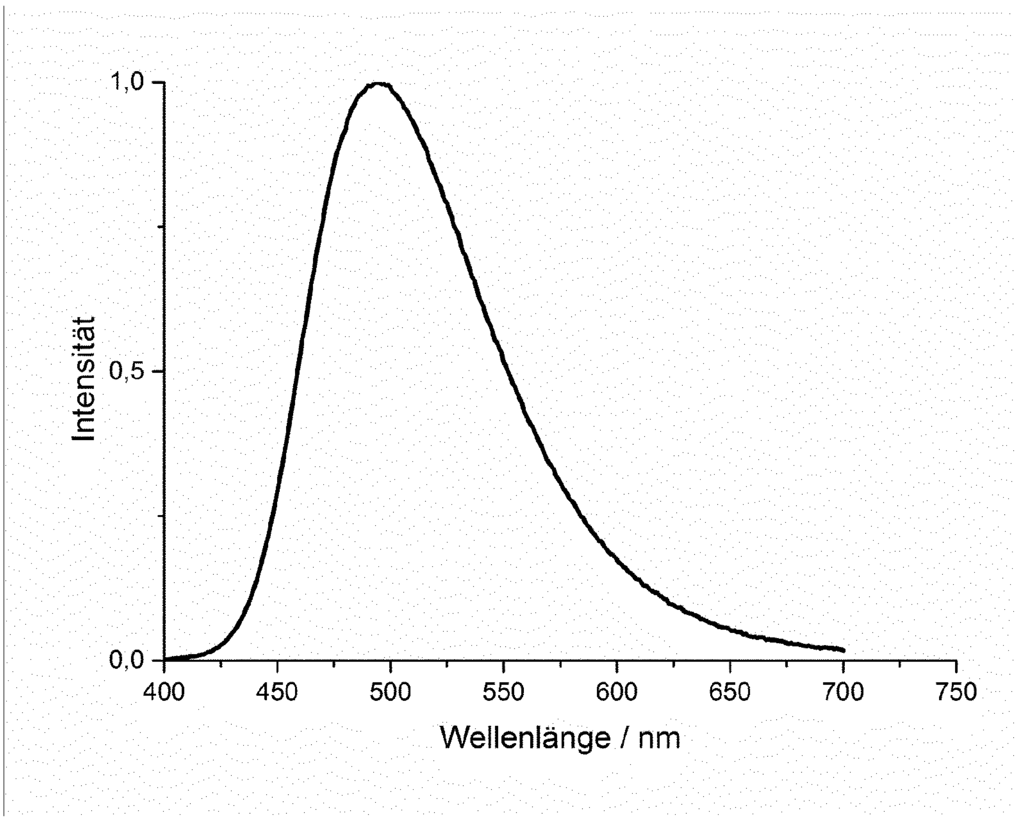

Figur 7

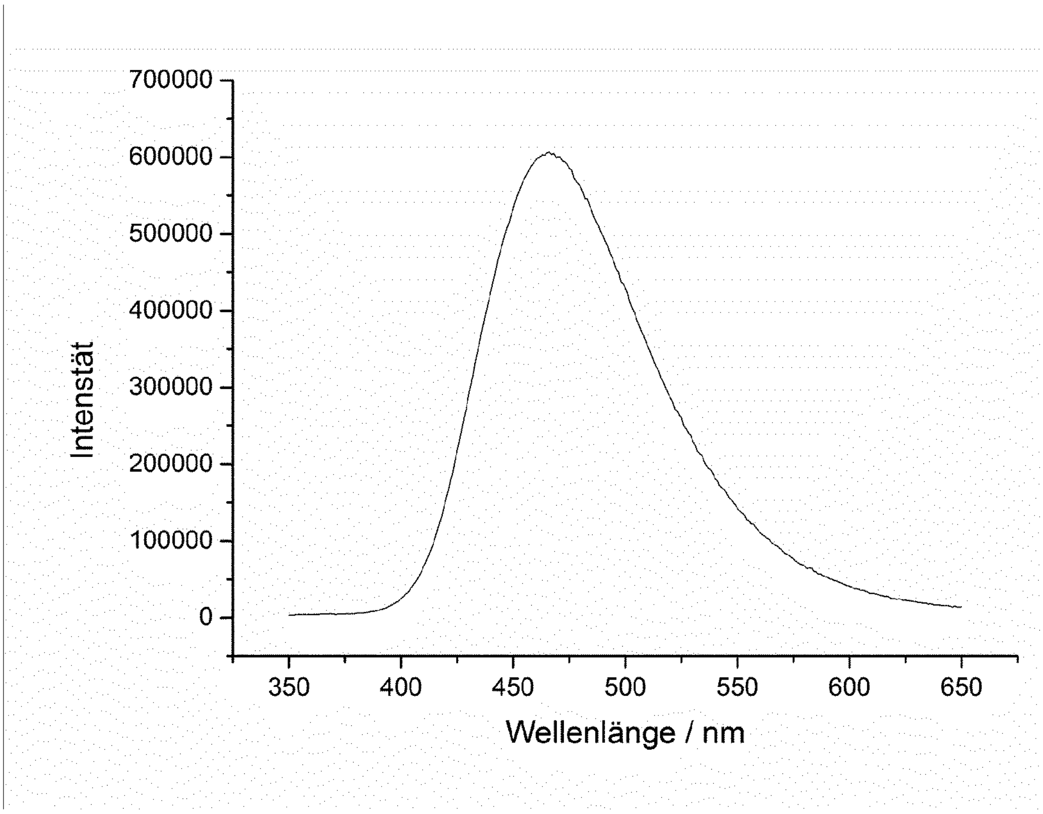


Figur 8

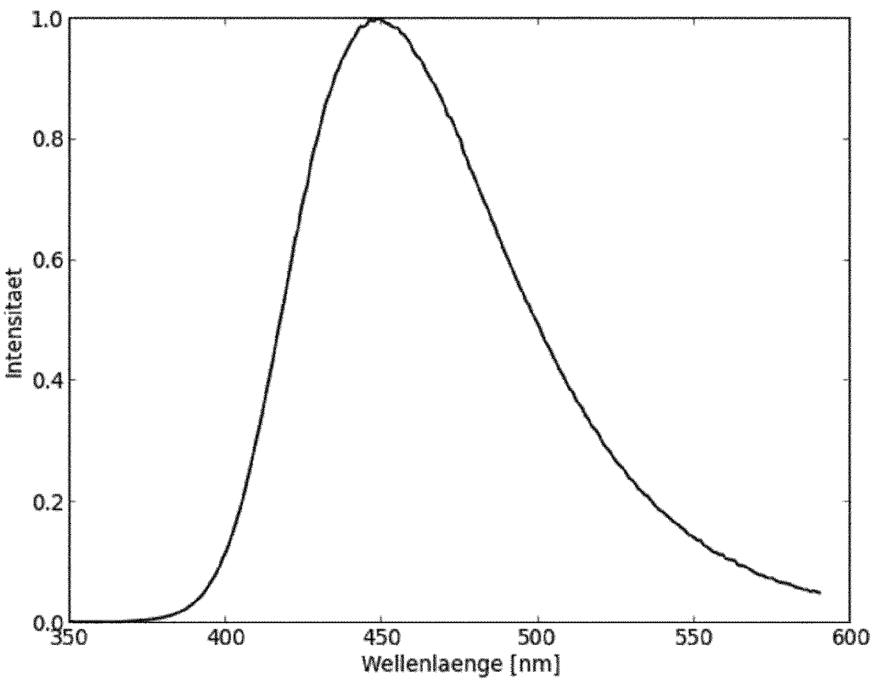

Figur 9

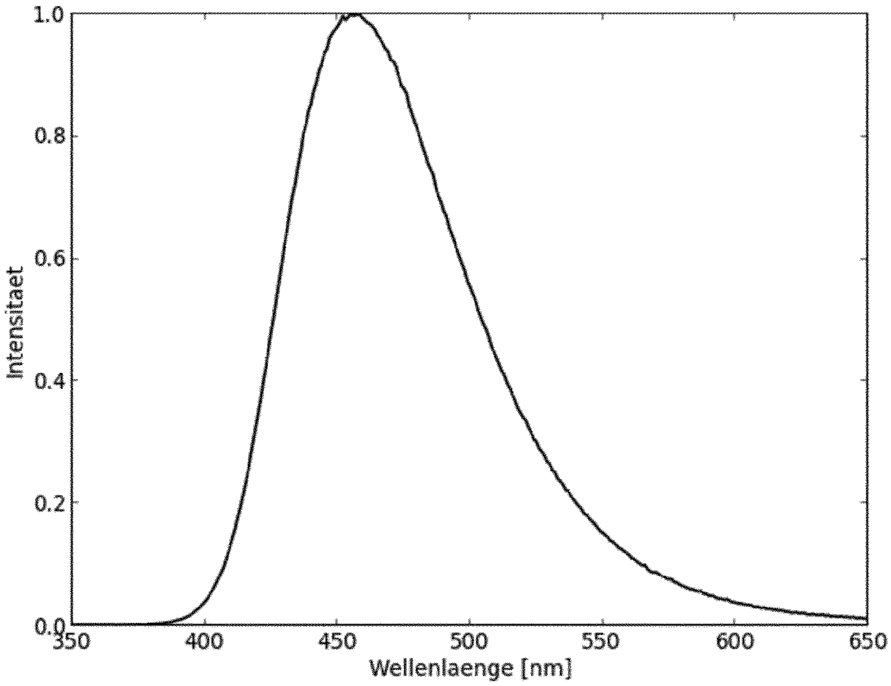


Figur 10

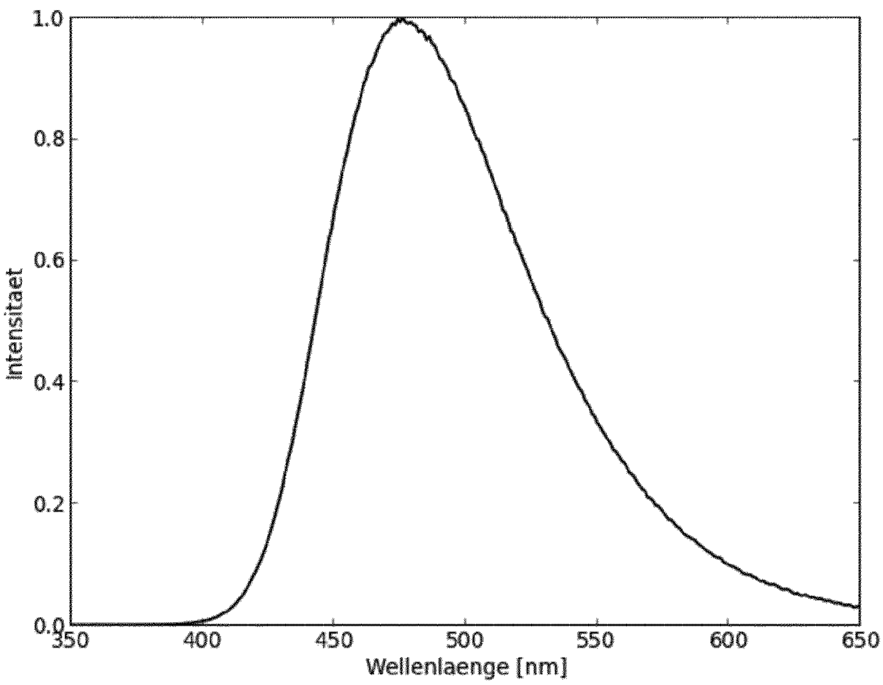

Figur 11

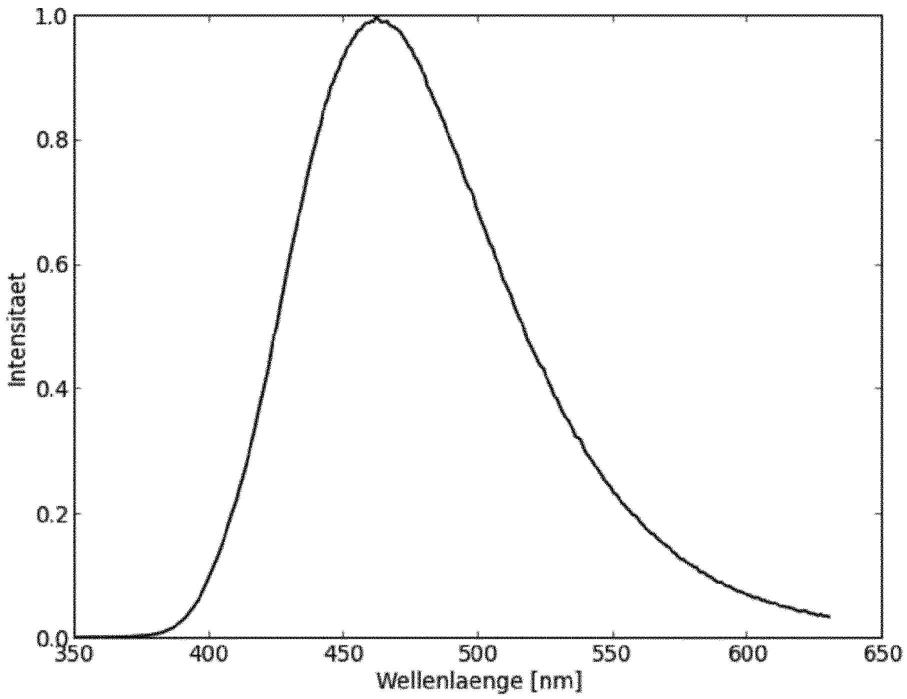


Figur 12

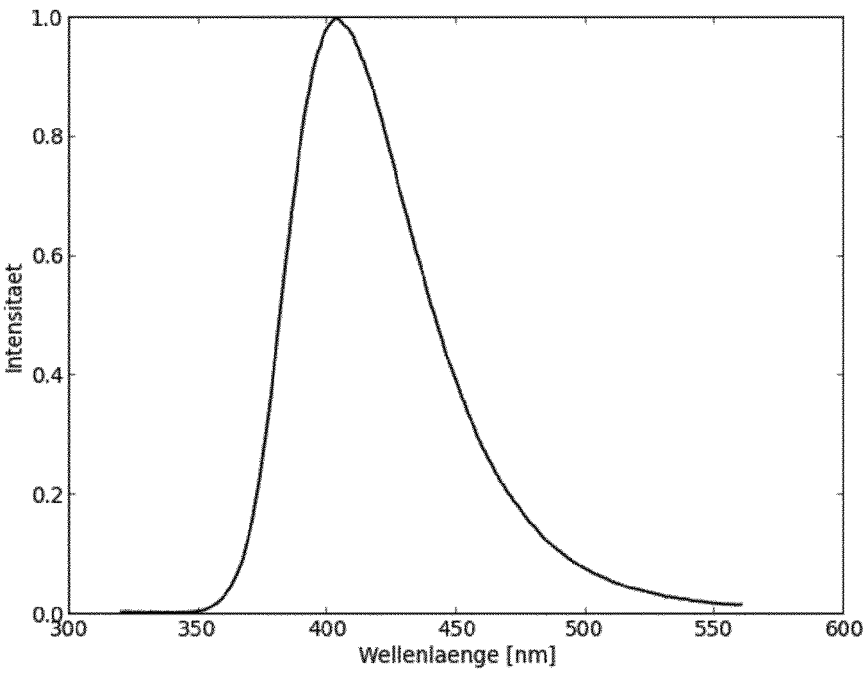

Figur 13

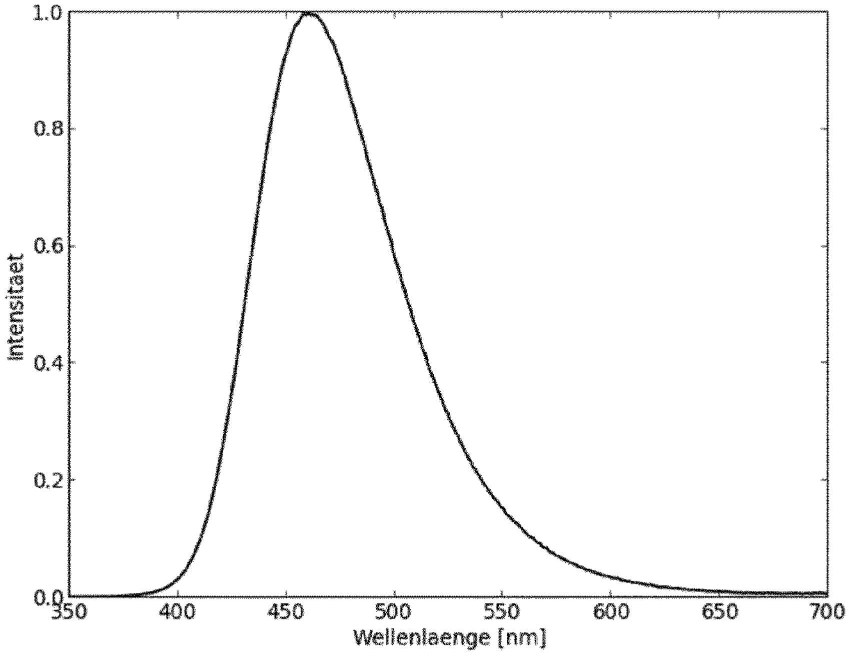


Figur 14

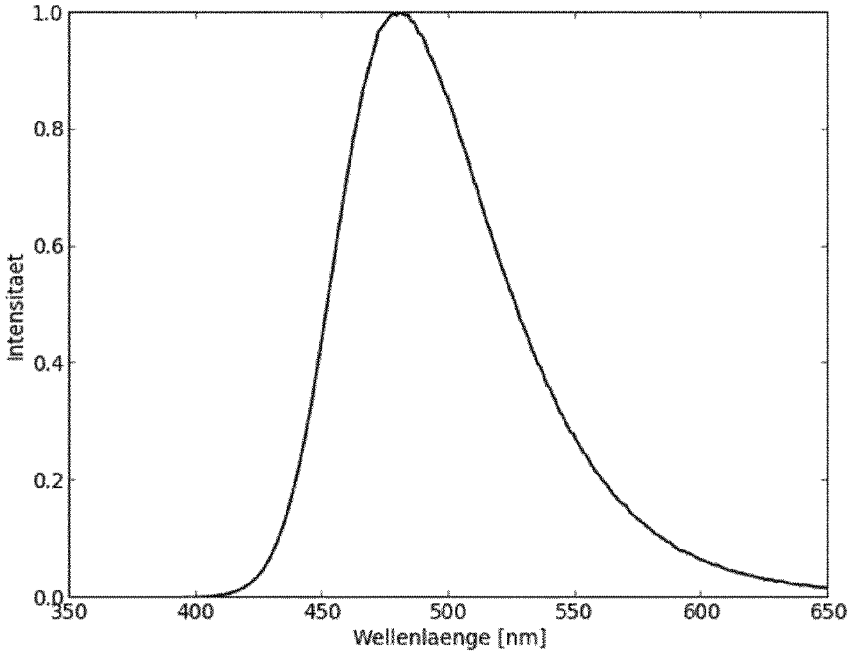


Figur 15

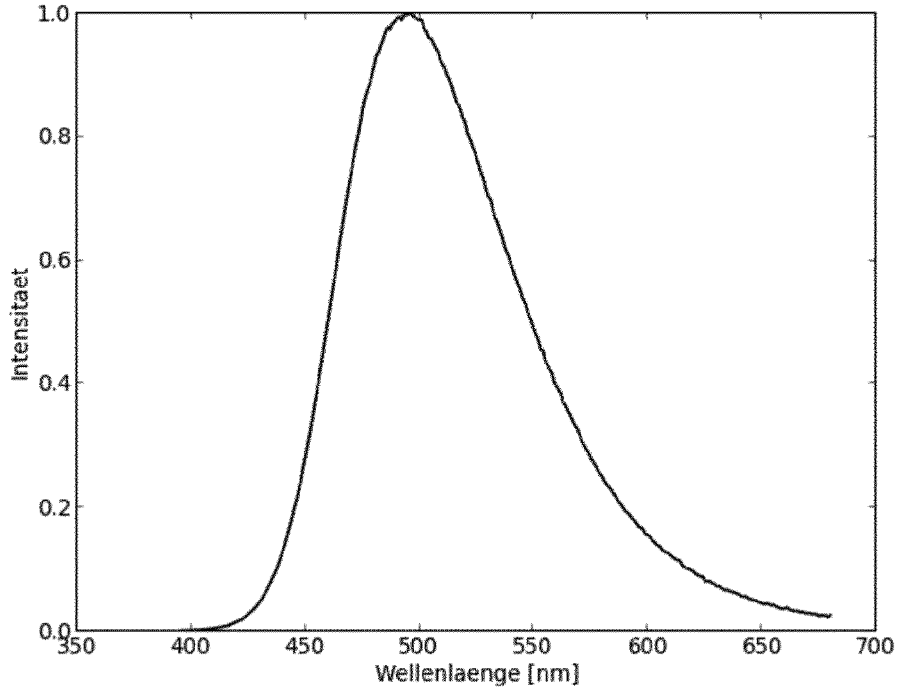


Figur 16

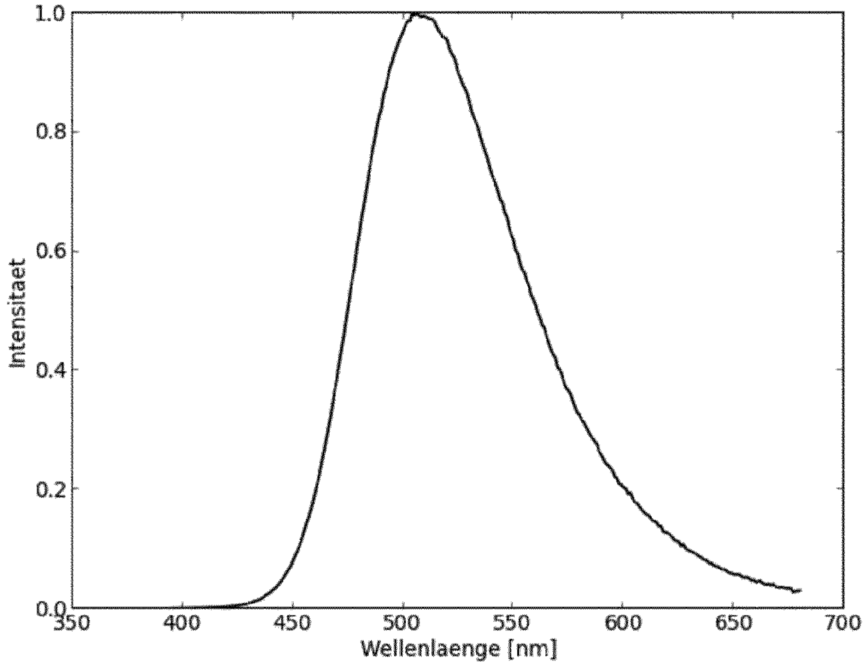


Figur 17

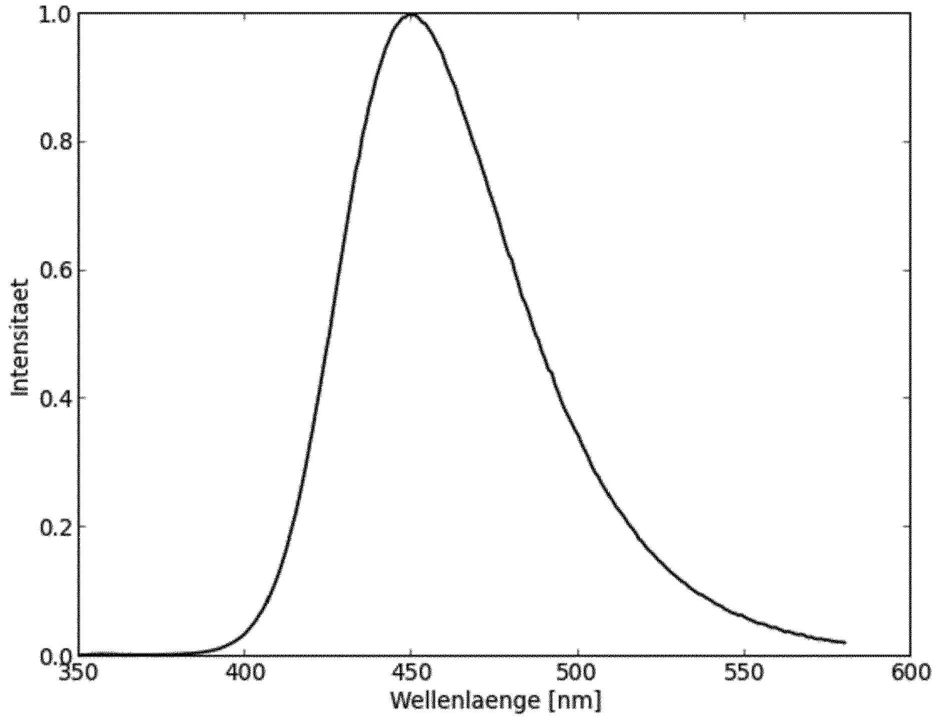
1.0
0.8
0.6
0.4
0.2
0.0
Intensitaet
350
400
450
500
550
600
Wellenlaenge [nm]

Figur 18

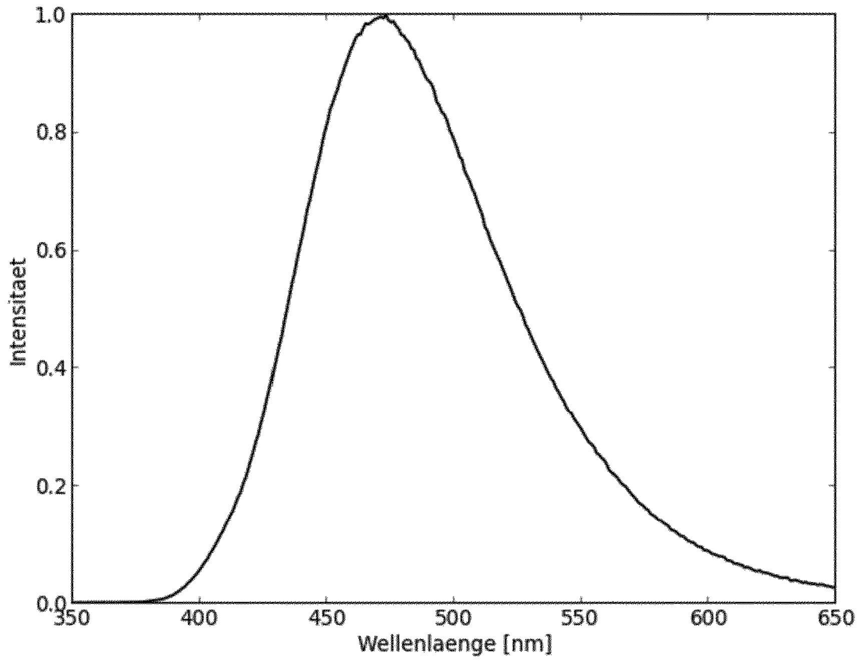
1.0
0.8
0.6
0.4
0.2
0.0
Intensitaet
350
400
450
500
550
600
650
Wellenlaenge [nm]

Figur 19

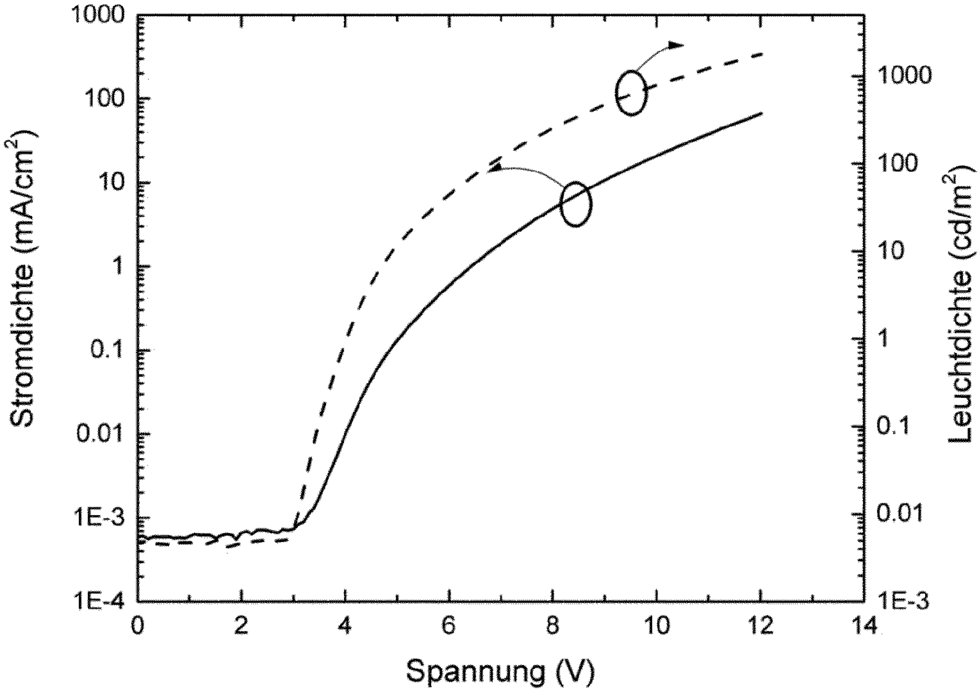


Figur 20

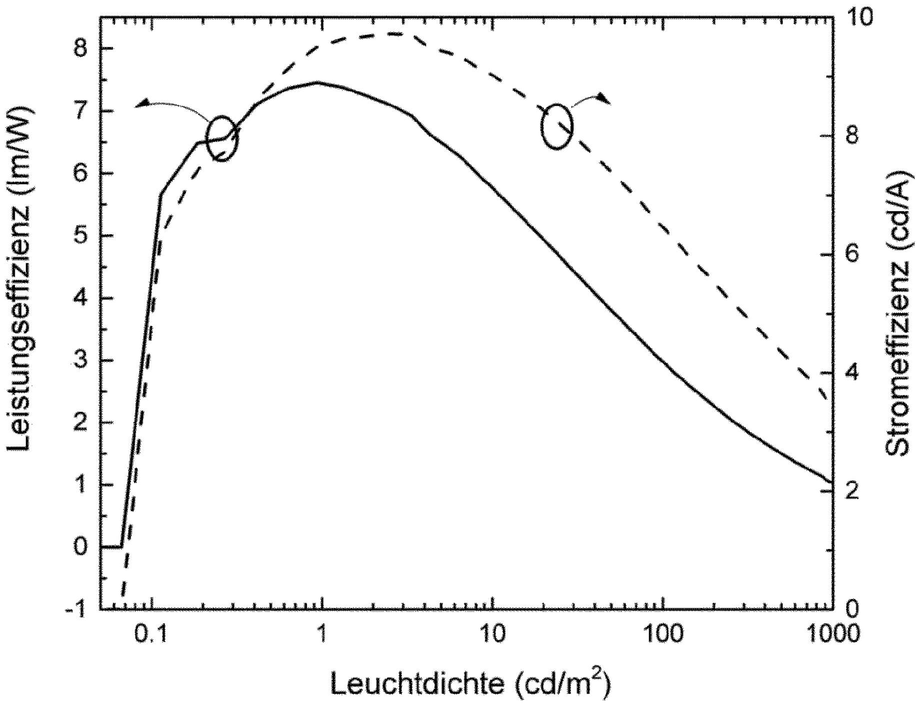

Figur 21

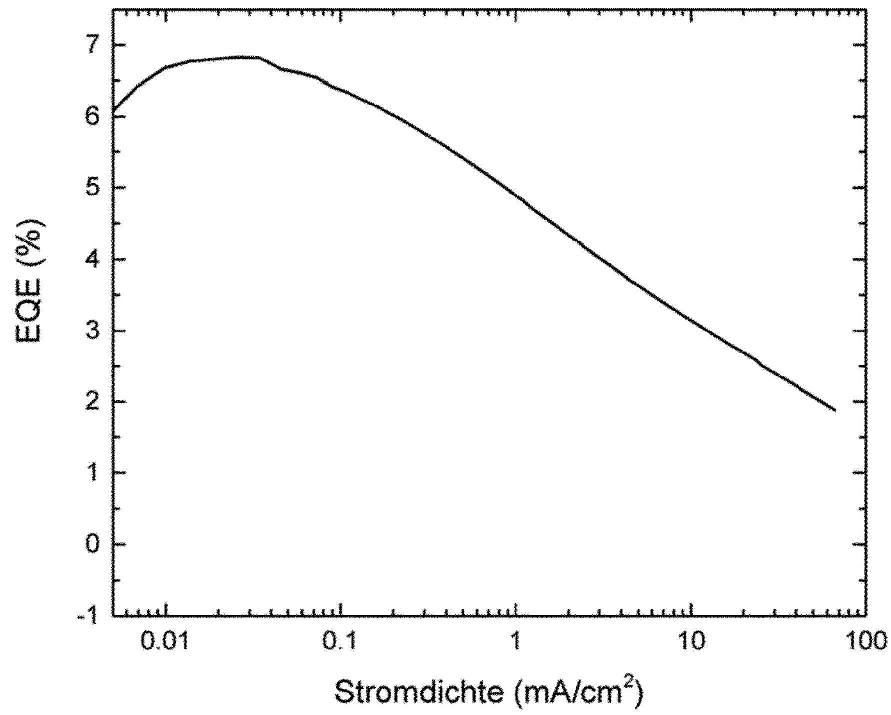


Figur 22

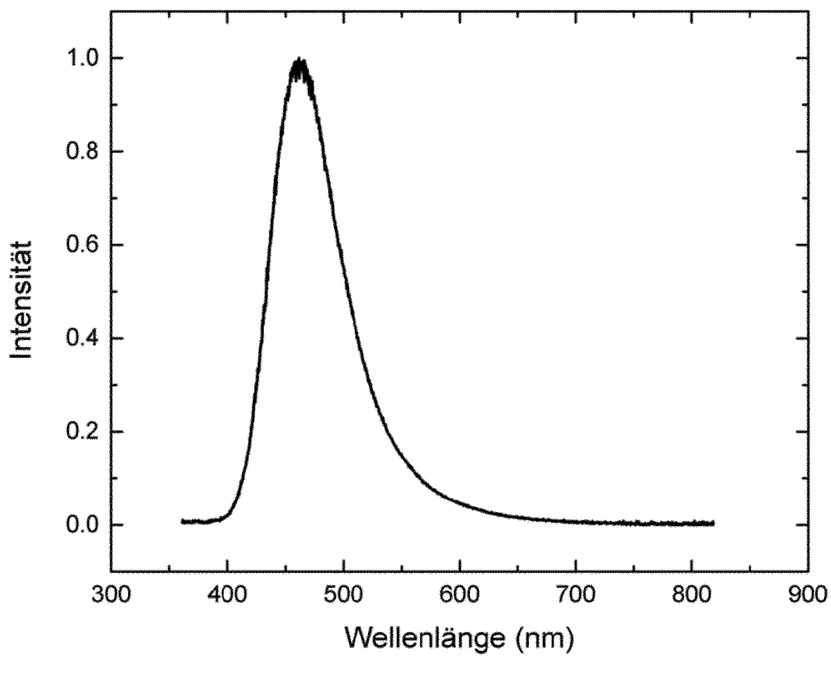

Europäisches Patentamt
European Patent Office
Office européen des brevets

# EUROPÄISCHER RECHERCHENBERICHT

Nummer der Anmeldung
EP 15 20 0813

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2014/146752 A1 (MERCK PATENT GMBH [DE]) 25. September 2014 (2014-09-25) * Zusammenfassung; Verbindung S97 * ----- | 1,3,5,6, 8-15 | INV. H01L51/30 ADD. H01L51/46 H01L51/54 |
| X | WO 2014/146750 A1 (MERCK PATENT GMBH [DE]) 25. September 2014 (2014-09-25) * Zusammenfassung; Verbindungen 96,97,100,103,123,124 * ----- | 1,3,8-15 | |
| A | RYOICHI ISHIMATSU ET AL: "Electrogenerated Chemiluminescence of Donor-Acceptor Molecules with Thermally Activated Delayed Fluorescence", ANGEWANDTE CHEMIE INTERNATIONAL EDITION, Bd. 53, Nr. 27, 22. Mai 2014 (2014-05-22), Seiten 6993-6996, XP55160406, ISSN: 1433-7851, DOI: 10.1002/anie.201402615 * das ganze Dokument * ----- | 1-15 | |
| A | JERZY HERBICH ET AL: "Phosphorescent intramolecular charge transfer triplet states", CHEMICAL PHYSICS LETTERS, Bd. 262, Nr. 5, 1. November 1996 (1996-11-01), Seiten 633-642, XP55213975, ISSN: 0009-2614, DOI: 10.1016/S0009-2614(96)01122-0 * das ganze Dokument * ----- -/-- | 1-15 | RECHERCHIERTE SACHGEBIETE (IPC) H01L |

Der vorliegende Recherchenbericht wurde für alle Patentansprüche erstellt

1

| Recherchenort | Abschlußdatum der Recherche | Prüfer |
|---|---|---|
| Den Haag | 19. Mai 2016 | Königstein, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 15 20 0813

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | HIROKI UOYAMA ET AL: "Highly efficient organic light-emitting diodes from delayed fluorescence", NATURE, Bd. 492, Nr. 7428, 12. Dezember 2012 (2012-12-12), Seiten 234-238, XP55048388, ISSN: 0028-0836, DOI: 10.1038/nature11687 * das ganze Dokument * ----- | 1-15 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Den Haag | 19. Mai 2016 | Königstein, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 15 20 0813

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-05-2016

| Im Recherchenbericht angeführtes Patentdokument | Datum der Veröffentlichung | Mitglied(er) der Patentfamilie | Datum der Veröffentlichung |
|---|---|---|---|
| WO 2014146752 A1 | 25-09-2014 | CN 105051014 A | 11-11-2015 |
| | | EP 2976329 A1 | 27-01-2016 |
| | | KR 20150132872 A | 26-11-2015 |
| | | US 2016072076 A1 | 10-03-2016 |
| | | WO 2014146752 A1 | 25-09-2014 |
| WO 2014146750 A1 | 25-09-2014 | CN 105073709 A | 18-11-2015 |
| | | EP 2976322 A1 | 27-01-2016 |
| | | KR 20150132873 A | 26-11-2015 |
| | | US 2016046563 A1 | 18-02-2016 |
| | | WO 2014146750 A1 | 25-09-2014 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010149748 A1, H. Yersin, U. Monkowius, T. Fischer, T. Hofbeck
- WO 2013161437 A1


### In der Beschreibung aufgeführte Nicht-Patentliteratur


- **Q. ZHANG et al.** *J. Am. Chem. Soc.,* 2012, vol. 134, 14706
- **M.E. THOMPSON et al.** *Chem. Mater.,* 2004, vol. 16, 4743
- **BECKE, A. D.** *Phys. Rev. A,* 1988, vol. 38, 3098-3100
- **PERDEW, J. P.** *Phys. Rev. B,* 1986, vol. 33, 8822-8827
- **SIERKA, M. ; HOGEKAMP, A. ; AHLRICHS, R. J.** *Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11
- TURBOMOLE V6.4 2012. TURBOMOLE GmbH, 1989